# EUROPEAN PATENT APPLICATION

(11) **EP 4 538 710 A1**
(43) Date of publication of application: **16.04.2025**
(21) Application number: 24205189.4
(22) Date of filing: 08.10.2024
(51) Int. Cl.: G01N 33/68

(54) **DERIVATIZATION REAGENT, DERIVATIZATION REAGENT KIT, AND METHOD FOR IMPROVING SENSITIVITY OF NITROGEN-CONTAINING COMPOUND IN MASS SPECTROMETRY**

(30) Priority: 11.10.2023 JP 2023176201
(71) Applicant: JEOL Ltd., Akishima-shi, Tokyo 196-8558 (JP)
(72) Inventor: FUKUZAWA, Seketsu, Akishima, 1968558 (JP); TAKIWAKI, Masaki, Akishima, 1968558 (JP); TAKAHASHI, Koji, Akishima, 1968558 (JP); KIKUTANI, Yoshikuni, Akishima, 1968558 (JP)
(74) Representative: Boult Wade Tennant LLP

(57) **Abstract**

The present invention provides a novel technique for improving the sensitivity of a nitrogen-containing compound in mass spectrometry. The present invention provides a derivatization reagent comprising a compound represented by Formula (1): RR'CO (1)
[in Formula (1), R and R' are each independently a hydrogen atom, a substituted or unsubstituted alkyl group having 1 to 11 carbon atoms, or a substituted or unsubstituted aryl group having 1 to 11 carbon atoms].

## Description

### Technical Field

The present invention relates to a derivatization reagent, a derivatization reagent kit, a method for improving the sensitivity of a nitrogen-containing compound in mass spectrometry, and the like.

### Background Art

Liquid chromatography-tandem mass spectrometry (LC-MS/MS) and matrix-assisted laser desorption-ionization mass spectrometry (MALDI-MS) are extremely powerful analytical techniques for quantifying trace amounts of metabolites or contents. In these analytical techniques, an analyte may be ionized. In the analysis in which an analyte is ionized, some analytes may be ionized inefficiently and thus cannot be detected, or detection accuracy may be insufficient for quantification in many cases. To address such problems, an analyte may be derivatized to improve ionization efficiency.

For example, Non-Patent Documents 1 and 2 disclose derivatization of lipophilic hormones containing no nitrogen atom (androgen or estrogen) with a derivatization reagent containing a nitrogen atom.

### Citation List

### Non-Patent Document

[Non-Patent Document 1] J. Chromatogr. B 2011, 879, 1159-1165.
[Non-Patent Document 2] Biomed. Chromatogr. 2021, 35, e5036.
[Non-Patent Document 3] Anal. Chem. 1997, 69, 137-144.
[Non-Patent Document 4] Rapid Commun Mass Spectrum 1999, 13. 1413-1422.
[Non-Patent Document 5] J. Am. Soc. Mass Spectrum 2017, 28, 1889-1900.

### Summary of the Invention

### Technical Problem

The present invention is intended to provide a novel technique for improving the sensitivity of a nitrogen-containing compound in mass spectrometry.

### Solution to Problem

The inventors of the present invention have found that, as described in Examples later, derivatization of a nitrogen-containing compound with an aldehyde or a ketone improves the sensitivity of the nitrogen-containing compound in mass spectrometry and have completed the present invention. The present invention will be described below.

The present invention provides a derivatization reagent comprising a compound represented by Formula (1) (suitably an aldehyde or a ketone):
RR'CO (1)
[in Formula (1), R and R' are each independently a hydrogen atom, a substituted or unsubstituted alkyl group having 1 to 11 carbon atoms, or a substituted or unsubstituted aryl group having 1 to 11 carbon atoms].

The present invention provides a derivatization reagent kit comprising the derivatization reagent and a reducing agent.

The present invention provides a method for improving the sensitivity of a nitrogen-containing compound in mass spectrometry. The method comprises derivatization of N-alkylating or N-arylating an amino group of a nitrogen-containing compound with the derivatization reagent kit (suitably a derivatization reagent comprising an aldehyde or a ketone) into a secondary amine or a tertiary amine.

The derivatization reagent may be used to N-alkylate or N-arylate an amino group of a nitrogen-containing compound.
The nitrogen-containing compound may be at least one compound selected from an amino acid, a peptide, and a catecholamine contained in a biological substance.
The derivatization reagent may be used to N-alkylate or N-arylate an amino group of a nitrogen-containing compound, and
   (a) when the nitrogen-containing compound is an amino acid or a peptide in a biological substance, R and R' in Formula (1) may each be independently a hydrogen atom or a substituted or unsubstituted alkyl group having 1 to 8 carbon atoms, or
   (b) when the nitrogen-containing compound is a catecholamine in a biological substance, R and R' in Formula (1) may each be independently a substituted or unsubstituted alkyl group having 2 to 9 carbon atoms.
The derivatization reagent or the derivatization reagent kit may be used for mass spectrometry.
The reducing agent may comprise at least one selected from a borane, a complex containing a borane, and a salt of a borohydride compound.

### Advantageous Effects of Invention

The present invention provides a novel technique for improving the sensitivity of a nitrogen-containing compound in mass spectrometry.

### Brief Description of Drawings

[Fig. 1] Fig. 1 shows a reaction scheme using an aliphatic or aromatic aldehyde or ketone and a reducing agent in the embodiments; in the reaction, an amine (1) and an aldehyde or ketone (2) are subjected to dehydration condensation to form an imine, and the formed imine is reduced with a reducing agent (into a secondary amine (3) or a tertiary amine (4)).
[Fig. 2] Fig. 2 shows chemical structural formulae of various amino acids (triiodothyronine (5), thyroxin (6), and homocysteine (7)) used in the embodiments.
[Fig. 3A] Fig. 3A shows various peptides (angiotensin I (8), oxytocin (9), glucagon (10), and adrenocorticotropic hormone (11)) used in the embodiments.
[Fig. 3B] Fig. 3B shows various peptides (insulin (12) and C-peptide (13)) used in the embodiments.
[Fig. 4] Fig. 4 shows various catecholamines (adrenaline (14), noradrenaline (15), and dopamine (16)) used in the embodiments.
[Fig. 5] Fig. 5 shows the results of ¹H-NMR analysis (upper view) and MS analysis (lower view) of a derivatized compound (T3-Me2) of an amino acid standard (T3) with an aldehyde reagent (formaldehyde).
[Fig. 6] Fig. 6 shows the results of ¹H-NMR analysis (upper view) and MS analysis (lower view) of a derivatized compound (T4-Me2) of an amino acid standard (T4) with an aldehyde reagent (formaldehyde).
[Fig. 7] Fig. 7 shows the results of ¹H-NMR analysis (upper view) and MS analysis (lower view) of a derivatized compound (T4-Bn2) of an amino acid standard (T4) with an aldehyde reagent (benzaldehyde).
[Fig. 8] Fig. 8 shows the results of ¹H-NMR analysis (upper view) and MS analysis (lower view) of a derivatized compound (HCys-iPr) of an amino acid standard (HCys) with a ketone reagent (acetone).
[Fig. 9] Fig. 9 shows the results of ¹H-NMR analysis (upper view) and MS analysis (lower view) of a derivatized compound (AI-Me2) of a peptide standard (AI) with an aldehyde reagent (formaldehyde).
[Fig. 10] Fig. 10 shows the results of ¹H-NMR analysis (upper view) and MS analysis (lower view) of a derivatized compound (AI-Et2) of a peptide standard (AI) with an aldehyde reagent (acetaldehyde).
[Fig. 11] Fig. 11 shows the results of ¹H-NMR analysis (upper view) and MS analysis (lower view) of a derivatized compound (AI-iPr) of a peptide standard (AI) with a ketone reagent (acetone).
[Fig. 12] Fig. 12 shows the results of ¹H-NMR analysis (upper view) and MS analysis (lower view) of a derivatized compound (OXT-Me2) of a peptide standard (OXT) with an aldehyde reagent (formaldehyde).
[Fig. 13] Fig. 13 shows the results of ¹H-NMR analysis (upper view) and MS analysis (lower view) of a derivatized compound (OXT-Et2) of a peptide standard (OXT) with an aldehyde reagent (acetaldehyde).
[Fig. 14] Fig. 14 shows the results of ¹H-NMR analysis (upper view A) and MS analysis (lower view B) of a derivatized compound (OXT-Pr2) of a peptide standard (OXT) with an aldehyde reagent (propionaldehyde).
[Fig. 15] Fig. 15 shows the results of ¹H-NMR analysis (upper view) and MS analysis (lower view) of a derivatized compound (OXT-iPr) of a peptide standard (OXT) with a ketone reagent (acetone).
[Fig. 16] Fig. 16 shows the results of ¹H-NMR analysis (upper view) and MS analysis (lower view) of a derivatized compound (GN-Me4) of a peptide standard (GN) with an aldehyde reagent (formaldehyde).
[Fig. 17] Fig. 17 shows the results of ¹H-NMR analysis (upper view) and MS analysis (lower view) of a derivatized compound (GN-Et2) of a peptide standard (GN) with an aldehyde reagent (acetaldehyde).
[Fig. 18] Fig. 18 shows the results of ¹H-NMR analysis (upper view) and MS analysis (lower view) of a derivatized compound (GN-Pr2) of a peptide standard (GN) with an aldehyde reagent (propionaldehyde).
[Fig. 19] Fig. 19 shows the results of ¹H-NMR analysis (upper view) and MS analysis (lower view) of a derivatized compound (GN-iPr) of a peptide standard (GN) with a ketone reagent (acetone).
[Fig. 20] Fig. 20 shows the results of ¹H-NMR analysis (upper view) and MS analysis (lower view) of a derivatized compound (INS-Me6) of a peptide standard (INS) with an aldehyde reagent (formaldehyde).
[Fig. 21] Fig. 21 shows the results of ¹H-NMR analysis (upper view) and MS analysis (lower view) of a derivatized compound (INS-Et5) of a peptide standard (INS) with an aldehyde reagent (acetaldehyde).

### Description of Embodiments

Preferred embodiments for carrying out the present technique will now be described. Embodiments described below are merely examples of typical embodiments of the present technique and do not narrow the scope of the technique. In the present description, percentages are expressed in terms of volume unless otherwise noted. The upper limits (or less) and the lower limits (or more) of numerical ranges (to) may be appropriately combined if desired. In the below explanations from "1." to "5." and the like, overlapping explanations of the technical features, constitutions, definitions, terms, methods, and the like of aldehydes, reducing agents, methods of improving the sensitivity in mass spectrometry, and the like may not be described. The explanations from "1." to "5." and the like may be applied to any technique of "1." to "5." or any embodiment, and each technical feature or the like may be appropriately adopted in any technique, any embodiment, or the like.

1. Description of the present invention

A compound having a nitrogen atom, such as a peptide and a protein, (hereinafter also referred to as a "nitrogen-containing compound") is more likely to be ionized than lipophilic hormones, and thus the derivatization of a nitrogen-containing compound is not a general method. Meanwhile, in the sequence analysis of proteins and amino acids, using a phosphonium compound having a phosphorus atom as a charge tag was reported (Non-Patent Document 3: Anal. Chem. 1997, 69, 137-144;

Non-Patent Document 4: Rapid Commun Mass Spectrum 1999, 13. 1413-1422.; Non-Patent Document 5: J. Am. Soc. Mass Spectrum 2017, 28, 1889-1900.). These documents, however, do not show whether the technique can be applied to quantitative determination.

Liquid chromatography-tandem mass spectrometry (LC-MS/MS) and matrix-assisted laser desorption-ionization mass spectrometry (MALDI-MS) are extremely powerful analytical techniques for quantifying trace amounts of metabolites or contents. Some compounds are, however, ionized inefficiently and thus cannot be detected or can be detected only at insufficient accuracy for quantification. Increasing the amount of a sample, such as a food or a supplement, can compensate the sensitivity, but the amount of a sample, such as a human metabolite, cannot be simply increased in many cases. To solve the problems, not only apparatus improvement such as higher sensitivity of a mass spectrometer but also modification (derivatization) of an analyte for higher ionization efficiency have been widely developed.

A typical biometabolite is an organic compound mainly comprising carbon, hydrogen, oxygen, and nitrogen, and an oxygen atom or a nitrogen atom, which has a lone electron pair, is likely to be added with a hydrogen ion (protonated) and has high ionization efficiency. In particular, a nitrogen atom has higher protonation properties than an oxygen atom, and a compound containing a nitrogen atom is more likely to be ionized than compounds containing no nitrogen atom. For a compound having no nitrogen atom, such as a lipophilic steroid hormone, a derivatization reagent is used to introduce a nitrogen atom for a higher ionization efficiency. However, a nitrogen-containing compound such as an amino acid, a peptide, a protein, and a catecholamine is likely to be ionized, and thus the sensitivity improvement by derivatization reaction has been difficult or has rarely been studied.

In LC-MS/MS, which is a conventional method of quantitative mass spectrometry, an analyte is ionized by electrospray ionization (ESI) or atmospheric pressure chemical ionization (APCI), and thus the easiness of ionization depends on the polarity (easiness of protonation) of the analyte and the organic solvent composition of a mobile phase that volatilizes in ionization. Different compounds have different structures and different polarities, and thus further improving the detection sensitivity involves optimizing the ionization efficiency for each compound. In LC-MS/MS, selective reaction monitoring (SRM) enables quantitative determination at higher sensitivity, and thus fragmentation to give a lower background noise is also important. Typically, in mass spectrometry in a positive ion mode, a nitrogen atom having a lone electron pair is easily protonated and has an ionization promoting effect. A nitrogen-containing compound such as an amino acid, a peptide, and a protein has a nitrogen atom, which is easily protonated, in the molecule and thus is more likely to be ionized than a non-nitrogen-containing compound.

For example, derivatization with such a strong cationic derivatization reagent as having a phosphonium group is likely to give a high background noise derived from the reaction reagent. In addition, derivatization increases the molecular weight, and thus the derivatized compound is unlikely to be ionized. Accordingly, the derivatization may offset or reduce the ionization efficiency improvement effect. In other words, if the sensitivity does not increase even after such effort as the derivatization reaction, direct measurement without derivatization is better. This has been generally believed in mass spectrometry of nitrogen-containing compounds. However, further improvements in the sensitivity are also needed for more accurate quantification of these nitrogen-containing compounds. There is therefore a demand for another method for detecting a nitrogen-containing compound at high sensitivity.

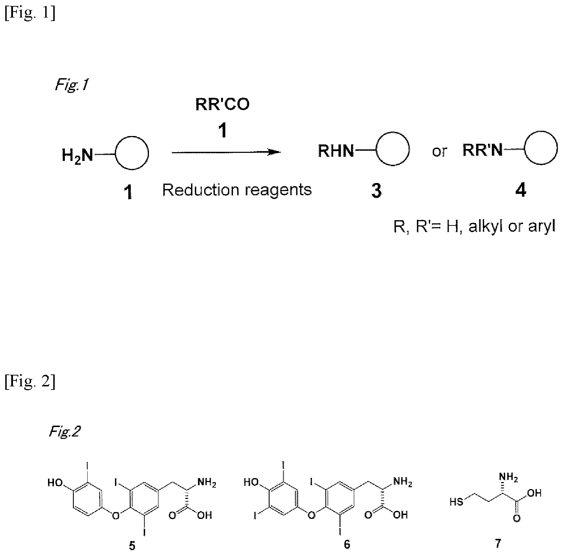

The inventors of the present invention have focused on an amino group as an ionization promoting group. Most of the amino acids, peptides, proteins, and catecholamines functioning in the living body are primary amines. Amines include primary amines, secondary amines, tertiary amines, and quaternary amines depending on the number of substituents, and the quaternary amine has a charge and is differentiated as an ammonium. Primary, secondary, and tertiary amines having a larger number of substituents typically have a larger basicity (an amine is more easily protonated) and have a larger hydrophobicity (an amine in a solvent containing a larger amount of an organic solvent is introduced to an ion source and thus is more easily ionized). An increase in basicity may be determined on the basis of an acid dissociation constant (pKa) as an index, and an increase in hydrophobicity may be determined on the basis of a hydrophobicity (ClogP) as an index. The hydrophobicity (ClogP) may be calculated with ChemDraw. In other words, the inventors of the present invention have assumed that secondarization or tertiarization of an amino group improves the ionization efficiency. In addition, for the secondarization or tertiarization of a primary amine, reductive amination reaction is used. The reaction may proceed by using an aldehyde or a ketone (for example, an aliphatic or aromatic aldehyde or ketone) and a reducing agent as follows: first, an amine (1) and an aldehyde or ketone (2) are subjected to dehydration condensation to form an imine; and the formed imine is reduced with a reducing agent (for example, Fig. 1). In the reaction, the reaction proceeds to form a secondary amine (3) or a tertiary amine (4) but fails to form a quaternary amine. Many types of aldehydes or ketones (for example, aliphatic aldehydes or ketones and aromatic aldehydes or ketones) are commercially available. Hence, the most suitable substituent can be selected according to the molecular weight or the hydrophobicity of an analyte. Accordingly, the ionization efficiency should be improved most suitably for each compound. In the conventional derivatization reaction, the residual reagent increases the background noise to result in a lower S/N. However, when a lower aliphatic aldehyde or ketone, which has a low boiling point, is used, the unreacted aldehyde or ketone is removed by concentration or nitrogen gas purge. In addition, a reducing agent (typically, a borohydride reagent) is converted into a watersoluble borate by hydrolysis reaction, and the borate is not adsorbed onto a reversed-phase column and is easily separated and removed, advantageously.

The present invention is preferably for detection and quantification of a trace metabolite at high sensitivity by mass spectrometry in the clinical examination field, and the technique can be used in a wide variety of fields for any apparatus regardless of the mass spectrometer manufacturer. In the future, to widely spread the quantification of a trace metabolite by mass spectrometry in the clinical examination field, it is desirable to produce a higher detection sensitivity regardless of the time, the place, and the apparatus from any manufacturer. The present invention enables the improvement in detection sensitivity, which bridges the difference between apparatuses from various manufacturers to help the spread.

The inventors of the present invention have found the followings, including Examples.
[1] The present invention provides a sensitivity improvement method in mass spectrometry by alkylating or arylating an amine into a secondary or tertiary amine.
[2] An analyte derivatized by the above derivatization reaction is more efficiently ionized by electrospray ionization (ESI), atmospheric pressure chemical ionization (APCI), or matrix-assisted laser desorption ionization (MALDI). The derivatized compound will be similarly ionized by other ionization methods. This enables the quantification of an analyte and the measurement or observation of the distribution (imaging) of the compound in a tissue or cell more accurately at a higher resolution.
[3] The derivatization reaction is carried out on nitrogen-containing compounds such as amino acids (typically, thyroid hormone), peptides (typically, oxytocin), and catecholamines (typically, dopamine) as a substrate. The substrate may be contained in a biological substance (such as blood, serum, plasma, urine, sweat, spinal fluid, hair, nails, and feces) and can be detected after an appropriate pretreatment.
[4] The alkylation or the arylation is preferably carried out with an aldehyde or a ketone and a reducing agent.
[5] The aldehyde or ketone is preferably an aliphatic or aromatic aldehyde having 1 to 12 carbon atoms. An aldehyde having a substituent such as a methyl group is also preferred.
[6] The reducing agent preferably comprises at least one selected from a borane, a complex containing a borane, a salt of a borohydride compound, and the like, and any reducing agent capable of exerting the same effect may be used.
[7] The present invention provides a derivatization reagent kit for mass spectrometry, and the kit comprises an aldehyde or a ketone and a reducing agent.

As apparent from Examples later, the technique is used to improve the sensitivity in mass spectrometry (suitably in LC-MS/MS analysis). As compared with a compound before derivatization, the sensitivity of an amino acid increases up to 2.5 times after derivatization; that of a peptide increases up to 17 times after derivatization; and that of a catecholamine increases up to 195 times after derivatization. By using the present technique, derivatization of a nitrogen-containing compound even in the living body such as blood plasma, serum, and urine improves the sensitivity of the nitrogen-containing compound in mass spectrometry.

2. First embodiment pertaining to the present invention (a derivatization reagent comprising an aldehyde or a ketone)

The first embodiment provides a derivatization reagent comprising an aldehyde or a ketone.

Another embodiment in the present invention may provide a derivatization reagent comprising <1> a compound represented by Formula (1): RR'CO (1) (R and R' are each independently a hydrogen atom, an alkyl group, or an aryl group), <2> a compound represented by Chemical Formula (2): RCHO (2) (R is a hydrogen atom, an alkyl group, or an aryl group) when R' in Formula (1) is a hydrogen atom, or <3> a compound represented by Formula (1) (R and R' are each independently an alkyl group or an aryl group) when neither R nor R' in Formula (1) is a hydrogen atom.

The derivatization reagent may be any of a composition, a derivatization composition, a derivatization agent, or a sensitivity increasing agent in mass spectrometry. With such a reagent, an analyte compound can be simply derivatized. In addition, derivatization improves the sensitivity (for example, the detection sensitivity or the measurement sensitivity) of an analyte. For example, the sensitivity in mass spectrometry should be improved.

In the first embodiment, an aldehyde or a ketone is preferably used as the derivatization reagent. For example, one or two or more compounds selected from the illustrative compounds of aldehydes or ketones described later may be used. The aldehyde or ketone is preferably used to derivatize a nitrogen-containing compound as an analyte and is preferably used to N-alkylate or N-arylate a nitrogen-containing compound. The derivatization reagent is preferably a mass spectrometry reagent or a reagent for mass spectrometry. For derivatization of an analyte, the aldehyde or ketone is preferably used in combination with at least the reducing agent described later.

By using the derivatization reagent in the first embodiment for an analyte, a derivatized (suitably N-alkylated or N-arylated) compound of the analyte is prepared. The N-derivatized analyte is subjected to measurement or detection, and the measurement or detection sensitivity of the analyte is improved.

### 2-1. Aldehyde or ketone

The aldehyde or ketone used in the present invention is not specifically limited. The aldehyde or ketone used in the present invention may be used as a derivatization reagent, a compound contained in a derivatization reagent kit, or a compound to be used in a derivatization reagent kit and may be used as the compound in production of such a reagent or such a reagent kit.

Examples of the aldehyde or ketone include aliphatic aldehydes or ketones, alicyclic aldehydes or ketones, aromatic aldehydes or ketones, and heterocyclic aldehydes or ketones. Any of them may be used, and one or two or more kinds selected from them may be used. Of them, an aliphatic aldehyde or ketone and/or an aromatic aldehyde or ketone is preferred.

The aldehyde or ketone may have any carbon number. From the viewpoint of sensitivity improvement, the lower limit of the carbon number is preferably 1, 2, or 3 or more, and the upper limit is preferably 20 or less, more preferably 15 or less, even more preferably 12 or less, and further preferably 10 or less. The carbon number is preferably an integer. The aldehyde or ketone used in the present invention may be a commercial product or may be produced in accordance with a known method for producing an aldehyde or a ketone.

The aldehyde or ketone used in a preferred embodiment in the present invention includes a compound represented by Formula (1): RR'CO (1) (in Formula (1), R and R' are each independently a hydrogen atom or a hydrocarbon group). The compound represented by Formula (1) may also be expressed as a compound represented by R-C(=O)-R', as needed. The hydrocarbon groups of R and R' in Formula (1) may each independently have a substituent or no substituent. The hydrocarbon group is preferably an alkyl group or an aryl group. The alkyl group may be a chain (straight-chain or branched-chain) alkyl group or a cyclic alkyl group. Preferably, R and R' in Formula (1) are each independently a hydrogen atom, a substituted or unsubstituted alkyl group having 1 carbon atom or 2 to 11 carbon atoms, or a substituted or unsubstituted aryl group having 1 carbon atom or 2 to 11 carbon atoms. In the compound represented by Formula (1), when one of R and R' is a hydrogen atom, the other may be a hydrogen atom or a hydrocarbon group. When neither R nor R' is a hydrogen atom, R and R' may each independently be a hydrocarbon group.

In Formula (1), examples of the hydrocarbon group of R and R' include, but are not necessarily limited to, aliphatic hydrocarbon groups, alicyclic hydrocarbon groups, and aromatic hydrocarbon groups. One or two or more kinds selected from them may be used.
The hydrocarbon group of R may have any carbon number. The lower limit of the carbon number is preferably 1 or 2 or more, and the upper limit is preferably 20 or less, more preferably 15 or less, even more preferably 12 or less, further preferably 10 or less, and furthermore preferably 9 or 8 or less.

Examples of the aliphatic hydrocarbon group include, but are not necessarily limited to, straight chain or branched chain aliphatic hydrocarbon groups (including straight chain or branched chain alkyl groups, alkenyl groups, and alkynyl groups) such as a methyl group, an ethyl group, a propyl group, an isopropyl group, a butyl group, an isobutyl group, a s-butyl group, a t-butyl group, a pentyl group, an isopentyl group, a hexyl group, a heptyl group, an octyl group, a nonyl group, a decyl group, a dodecyl group, a tetradecyl group, a hexadecyl group, an octadecyl group, and an allyl group. Such a group may be either a straight chain group or a branched chain group. Of them, a straight chain or branched chain alkyl group is preferred.

Of them, a substituted alkyl group (for example, an arylalkyl group) having a substituent (for example, an aryl group) or an unsubstituted alkyl group is preferred. One or two or more kinds selected from them may be used. The aryl group may be identical to the aryl group described later, and examples include a phenyl group.

The aliphatic hydrocarbon group may have any carbon number, but the carbon number is preferably 1 or 2 to about 20 and more preferably 1 or 2 to about 10. One or two or more kinds selected from them may be used.

Examples of the alicyclic hydrocarbon group include, but are not necessarily limited to, aliphatic monocyclic hydrocarbon groups (including cycloalkyl groups and cycloalkenyl groups) such as a cyclopropyl group, a cyclobutyl group, a cyclopentyl group, a cyclohexyl group, a cyclohexenyl group, a cyclooctyl group, a cyclodecyl group, and a cyclododecyl group; and bridged cyclic hydrocarbon groups. Typical examples of the bridged ring in the bridged cyclic hydrocarbon group include an adamantane ring, a norbornane ring, and a norbornene ring. To the alicyclic moiety, an aromatic ring may be condensed. Of them, a cycloalkyl group (cyclic alkyl group) is preferred. The alicyclic hydrocarbon group may have any carbon number but preferably has about 3 to 20 carbon atoms. One or two or more kinds selected from them may be used.

Examples of the aromatic hydrocarbon group include aryl groups including monocyclic aromatic hydrocarbon groups such as a phenyl group, a tolyl group, and an o-xylyl group; and polycyclic aromatic hydrocarbon groups such as a naphthyl group, and the aryl group may have a substituent such as an alkyl group. The aromatic ring may be condensed with a non-aromatic ring. The aromatic hydrocarbon group may have any carbon number but preferably has about 6 to 20 carbon atoms. Of them, a substituted aryl group (for example, an alkyl aryl group) having a substituent (preferably an alkyl group) or an unsubstituted aryl group is preferred. One or two or more kinds selected from them may be used. The alkyl group may be identical with the alkyl group described above, and examples include a methyl group and an ethyl group.

The hydrocarbon group may be a substituted or unsubstituted hydrocarbon group. The substituted hydrocarbon group may have, as the "substituent", various substituents (for example, a substituent having 1 to 3 carbon atoms), such as a halogen atom, an oxo group, a hydroxyl group, a substituted oxy group (for example, an alkoxy group, an aryloxy group, and an acyloxy group), a carboxyl group, a substituted oxycarbonyl group, a substituted or unsubstituted carbamoyl group, a cyano group, a nitro group, a substituted or unsubstituted amino group, a straight chain or branched chain alkyl group (for example, an alkyl group such as a methyl group and an ethyl group), a cyclic alkyl group, an aryl group (for example, a phenyl group and a naphthyl group), and a heterocyclic group. Of them, the substituent is preferably a hydrocarbon group (an unsubstituted or substituted hydrocarbon group). More specifically, the substituent is preferably selected from, for example, unsubstituted alkyl groups (the alkyl group is a straight chain alkyl group, a branched chain alkyl group, or a cyclic alkyl group), aryl groups having an unsubstituted aryl group, and aryl groups having an unsubstituted alkyl group. The substituent may have the same carbon number as above. One or two or more kinds selected from them may be used.

Preferred examples of the aliphatic aldehyde used in the present invention include aliphatic saturated aldehydes such as formaldehyde, acetaldehyde, propionaldehyde, butyraldehyde, isobutyraldehyde, valeraldehyde (pentanal), isovaleraldehyde, pivalaldehyde, hexanal, heptanal, octanal, nonanal, decanal, dodecanal, tetradecanal, hexadecanal, and octadecanal; aliphatic dialdehydes such as glyoxal and succindialdehyde; and aliphatic unsaturated aldehydes such as acrolein, crotonaldehyde, and pentenal. One or two or more kinds selected from them may be used. Preferred examples of the aliphatic ketone include acetone, methyl ethyl ketone, and 3-pentanone.

Preferred examples of the alicyclic aldehyde used in the present invention include cyclopropanecarboxaldehyde, cyclopentanecarbaldehyde, cyclohexanecarbaldehyde, cyclooctanecarbaldehyde, cyclododecanecarbaldehyde, and 5-norbornene-2-carboxaldehyde. One or two or more kinds selected from them may be used.

Preferred examples of the aromatic aldehyde used in the present invention include benzaldehyde, o-, m-, p-tolualdehydes, salicylaldehyde, cinnamaldehyde, α-, β-naphthaldehydes, phthalaldehyde, terephthalaldehyde, and isophthalaldehyde. One or two or more kinds selected from them may be used.

As preferred examples of the aldehyde used in the present invention, for example, one or two or more kinds selected from aliphatic aldehydes such as formaldehyde, acetaldehyde, propionaldehyde, butyraldehyde, isobutyraldehyde, valeraldehyde (pentanal), isovaleraldehyde, pivalaldehyde, hexanal, and heptanal; alicyclic aldehydes such as cyclopropanecarboxaldehyde; and aromatic aldehydes such as benzaldehyde is preferred. Of them, aliphatic aldehydes are preferred. Of the exemplary aliphatic aldehydes, one or two or more kinds selected from formaldehyde, acetaldehyde, propionaldehyde, butyraldehyde, isobutyraldehyde, valeraldehyde (pentanal), isovaleraldehyde, hexanal, and heptanal is more preferred.

As preferred examples of the ketone used in the present invention, for example, one or two or more kinds selected from aliphatic ketones including acetone, methyl ethyl ketone, and 3-pentanone is preferred. Of the exemplary aliphatic ketones, acetone is more preferred.

### 2-2. Analyte

In the present invention, the analyte to be derivatized is not specifically limited but is preferably a nitrogen-containing compound. The analyte may be a substrate, a target substance to be derivatized, or a target substance to be subjected to mass spectrometry.

The nitrogen-containing compound used in the present invention preferably has at least one primary amine (HHN-) in the molecular structure of the nitrogen-containing compound. In the molecular structure, the nitrogen-containing compound preferably has at least one aryl group or methylene group. The aryl group or the methylene group may be unsubstituted or substituted, and examples include an aryl group having an alkyl group. The alkyl group may be unsubstituted or substituted, and examples include chain (straight chain, branched chain) alkyl groups and cyclic alkyl groups. The alkyl group may have any carbon number but, for example, has about 2 to 4 carbon atoms. Examples of the substituent include, but are not necessarily limited to, an alkyl group, an iodine atom, a hydroxy group, a carbonyl group, and an amino group. One or two or more kinds may be selected from them.

The nitrogen-containing compound is preferably one or two or more kinds selected from amino acids, peptides, catecholamines, proteins, and the like and may be a synthetic compound or a natural compound. Examples include clinical examination samples (for example, biological substances, animal or plant tissues, and animal or plant cells), environmental hygiene test samples, and products (food and drink products, pharmaceutical products, quasi-drugs, cosmetics, and chemical agents).

As a more preferred example, for example, one or two or more kinds selected from biological substances, organic synthetic compounds, and separation and purification products is preferred. By applying the present technique to a nitrogen-containing compound that is contained in a biological substance and is typically difficult to measure or detect, after an appropriate pretreatment, the nitrogen-containing compound is satisfactory derivatized, and the product is suitable as a derivatized compound for mass spectrometry. Hence, the present invention is preferably used to measure or detect a biological substance and is more preferably used for a nitrogen-containing compound contained in a biological substance as the analyte.

Examples of the nitrogen-containing compound include, but are not necessarily limited to, amino acids (such as triiodothyronine, thyroxin, and homocysteine), peptides (such as angiotensin I, oxytocin, glucagon, adrenocorticotropic hormone, insulin, and C-peptide), and catecholamines (such as adrenaline, noradrenaline, dopamine, and levodopa). One or two or more kinds selected from them may be used.

In the present invention, the analyte is preferably one or two or more kinds selected from amino acids, peptides, and catecholamines contained in a biological substance.

When the analyte is an amino acid (preferably an amino acid in a biological substance), the number of primary amines (HHN-) in the molecular structure is preferably 1 to 2 and more preferably 1, and the number of aryl groups in the molecular structure is preferably 0 to 3, more preferably 0 to 2, and even more preferably 2. The amino acid as the analyte preferably has a molecular weight of 50 to 1,000 and more preferably 100 to 800. The molecular weight is calculated as the sum of atomic weights in the molecule. The amino acid as the analyte has a hydrophobicity (ClogP) of -4 to 16 and more preferably -2 to 10.

When the analyte is a peptide (preferably a peptide in a biological), the number of primary amines (HHN-) in the molecular structure is not specifically limited. The lower limit of the number of primary amines is preferably 1 or more and more preferably 2 or more, and the upper limit is preferably 30 or less, more preferably 20 or less, even more preferably 15 or less, and further preferably 10 or less. When the analyte is a peptide, the number of aryl groups in the molecular structure is not specifically limited. The lower limit of the number of aryl groups is preferably 1 or more and more preferably 2 or more, and the upper limit is preferably 30 or less, more preferably 20 or less, even more preferably 15 or less, and further preferably 10 or less. As for the molecular weight of the peptide as the analyte compound, the lower limit is preferably 100 or more and more preferably 200 or more, and the upper limit is preferably 10,000 or less, more preferably 9,000 or less, even more preferably 8,000 or less, and further preferably 6,000 or less. As for the hydrophobicity (ClogP) of the peptide as the analyte compound, the lower limit is preferably -4 or more and more preferably -2 or more, and the upper limit is preferably 10 or less, more preferably 8 or less, even more preferably 6 or less, and further preferably 4 or less.

From the viewpoint of easily improving the sensitivity of an analyte in the technique, of the above amino acids, triiodothyronine, thyroxin, and homocysteine are preferred; of the above peptides, angiotensin I, oxytocin, glucagon, insulin, and C-peptide are preferred; and of the above catecholamines, adrenaline, noradrenaline, and dopamine are preferred. One or two or more kinds selected from them may be used.

In a preferred embodiment of the present invention, (a) when the nitrogen-containing compound is an amino acid or a peptide, R and R' of RR'CO in Formula (1) are preferably each independently a hydrogen atom or a substituted or unsubstituted alkyl group having 1 to 8 carbon atoms. Alternatively, (b) when the nitrogen-containing compound is a catecholamine, R and R' of RR'CO in Formula (1) are preferably each independently a substituted or unsubstituted alkyl group having 2 to 9 carbon atoms.

In a more preferred embodiment, (a) when the nitrogen-containing compound is an amino acid or a peptide, R and R' of RR'CO in Formula (1) are preferably each independently a hydrogen atom or a substituted or unsubstituted alkyl group having 1 to 3 carbon atoms, and more preferably a hydrogen atom or a straight chain alkyl group having 1 to 2 carbon atoms. Even more preferably, R and R' are independently one or two or more kinds selected from a hydrogen atom, a methyl group, an ethyl group, a propyl group, and the like. The analyte in a sample is preferably an amino acid and/or a peptide in a biological substance.

(a) When the nitrogen-containing compound is an amino acid or a peptide, each carbon number of R and R' in Formula (1) is preferably 0 to 5, more preferably 4 or less or 3 or less, and even more preferably 0, 1, or 2. When each carbon number of R and R' is 0, R and R' are preferably a hydrogen atom. The aldehyde or ketone is more preferably an aldehyde or ketone (preferably having 1 to 3 carbon atoms) having a straight chain alkyl group having 1 to 2 carbon atoms or a hydrogen atom so as to give a secondary or tertiary compound in which the primary or secondary amino group of an analyte compound is derivatized into N-methyl, N-ethyl, N-propyl, N-isopropyl, or the like. The analyte in a sample is preferably an amino acid and/or a peptide in a biological substance.

(b) When the nitrogen-containing compound is a catecholamine, R of RR'CO in Formula (1) is preferably a substituted or unsubstituted alkyl group having 3 to 9 carbon atoms. The carbon number of the alkyl group is preferably 4 to 8 and more preferably 4 to 6. The alkyl group is preferably an unsubstituted alkyl group, and R' is preferably a hydrogen atom. More preferably, R is preferably one or two or more kinds selected from a butyl group, an isobutyl group, a pentyl group, a hexyl group, a phenyl group, and the like. Of them, a hexyl group is more preferred. The analyte in a sample is preferably a catecholamine in a biological substance.

The biological substance may be derived from any material and from any of animals, plants, microorganisms, and the like. The biological substance is not specifically limited. An animal biological substance is preferred, and examples include blood, serum, plasma, urine, sweat, spinal fluid, hair, nails, and feces. One or two or more kinds selected from them may be used. The animal is not specifically limited but is one or two or more kinds selected from mammals (such as humans, mice, dogs, cats, rabbits, horses, pigs, and cattle), birds (such as fowls and pigeons), reptiles, amphibians, and the like. From the viewpoint of medical examination or the like, humans, pet animals (such as cats and dogs), competing animals (such as horses, dogs, and rabbits), and livestock/poultry (such as pigs, cattle, and fowls) are preferred. A substance such as a biological substance sampled from a living thing such as an animal may be used as the analyte, and the sampled substance may be pretreated before derivatization.

By the derivatization reagent used in the present invention, a primary amine contained in an analyte is converted into a secondary amine or a tertiary amine. More specifically, by the derivatization reagent used in the present invention, a primary amine contained in an analyte is N-alkylated or N-arylated. In other words, an amino group of an analyte is subjected to secondarization or tertiarization, and accordingly the ionization efficiency is improved. When an analyte is derivatized into a secondary amine or a tertiary amine, and the derivatized compound is subjected to mass spectrometry, the intensity or the area of the parent ion peak increases as compared with the intensity or the area of the parent ion peak of the analyte compound before derivatization in mass spectrometry. Hence, the sensitivity in mass spectrometry increases.

The separation and purification method of the analyte in a sample in the present invention is not specifically limited, and a separation and purification method appropriate for the type of a sample may be used. The separation and purification method of the biological substance, the derivatized compound, or the like is not specifically limited, and a known separation and purification method may be used for a substance such as a biological substance, the derivatized compound, or the like. Examples of the separation and purification method used in the present invention include, but are not necessarily limited to, centrifugation, precipitation (such as acetonitrile precipitation and methanol precipitation), column chromatography (such as ion exchange chromatography, gel filtration chromatography, hydrophobic chromatography, affinity chromatography, normal-phase chromatography, reversed-phase chromatography, and metal chelate chromatography), ultrafiltration, and antibody. These methods may be used singly or in combination of two or more of them. The substance such as an ion exchanger and a metal chelate used as the support of column chromatography may be used for a separation and purification method other than the column chromatography. The separation and purification method are preferably carried out before the derivatization reaction, but the separation and purification method may be carried out after the derivatization reaction.

The ion exchanger is not specifically limited, and either an anion exchanger or a cation exchanger may be used. According to the analyte sampled or captured from a sample such as a biological substance, any of a strong anion exchanger, a weak anion exchanger, a strong cation exchanger, and a weak cation exchanger may be used. Examples of the hydrophobic group used in a hydrophobic column chromatography containing a substance having a hydrophobic group include alkyl groups having 4 to 20 carbon atoms and a phenyl group. Examples of the metal chelate include a metal chelate having a metal ion such as Cu²⁺, Zn²⁺, Ni²⁺, Ca²⁺, Co²⁺, and Mg²⁺, to which a molecule, an ion, or the like referred to as a ligand may be bonded. Examples of the antibody include an antibody specific to the analyte. One or two or more kinds selected from them may be used.

For example, a sample such as a biological substance is loaded onto an ion exchange column, and a solvent is used to wash the column and to elute the analyte. The eluate containing the target substance is concentrated to dryness. From a sample such as a biological substance, an amino acid, a peptide, a catecholamine, or the like may be extracted by a known extraction method. This enables separation and purification of a target substance to be derivatized from a sample such as a biological substance, and this further improves the derivatization efficiency.

For example, an analyte contained in a sample such as a biological substance may be captured by using a support that is appropriately selected from supports typically used for separation and purification. As the support, one or two or more kinds selected from substances having ionic bonding properties, substances having affinity, substances having chemical interaction such as hydrophobic interaction (for example, a hydrophobic group), and the like may be used.

In the present invention, according to the molecular weight and the hydrophobicity of an analyte, the best suited derivatization reagent capable of improving the sensitivity in mass spectrometry may be selected from the aldehydes and ketones used in the present invention, advantageously. Moreover, many of the aldehydes and ketones used in the present invention are commercially available, and thus an aldehyde or ketone derivatization reagent capable of further improving the sensitivity is easily found, advantageously.

In the present invention, an optional component may be appropriately mixed with the derivatization reagent. For example, one or two or more kinds selected from reducing agents, acids, bases, and the like may be used, but the base is not necessarily used.

3. Second embodiment pertaining to the present invention (derivatization reagent kit)

The second embodiment provides a derivatization reagent kit comprising a reagent containing an aldehyde or a ketone, and the reagent containing an aldehyde or a ketone may be a derivatization reagent containing an aldehyde or a ketone. Another embodiment may provide a derivatization reagent kit comprising a reagent containing a compound represented by Formula (1) (RR'CO (1)) (where R and R' may be identical or different) or containing, when R' in Formula (1) is a hydrogen atom, a compound represented by Chemical Formula (2) (RCHO (2)).

The reagent kit in the second embodiment preferably further comprises a reducing agent, and the reducing agent may be a derivatization reagent or composition containing a reducing agent. The kit may be a combination or a set, which may be a product such as a kit product. The present invention may be a single-pack composition comprising, for example, an aldehyde or ketone and a reducing agent together or may be a multi-pack reagent kit such as a two-pack kit, for example, comprising an aldehyde or ketone in a container and comprising a reducing agent in another container.

A more preferred embodiment in the second embodiment is a derivatization reagent kit comprising a first reagent containing an aldehyde or ketone and a second reagent containing a reducing agent. For derivatization, an acid such as acetic acid is preferably used. For example, the derivatization reagent kit may further comprise a third reagent containing an acid or an acid. Each component may be a solution in a solvent. Each component may be used in a derivatization method, a derivatization reagent, or a derivatization reagent kit and may be used as a compound to produce such a reagent or a reagent kit. The reagent kit may further comprise, in addition to the aldehyde or ketone, the reducing agent, and the acid, a fourth derivatization reagent containing a base (such as ammonia, an alkali metal hydroxide, and an alkali earth metal hydroxide). In the present embodiment, a base reagent for derivatizing an analyte is not necessarily added to the derivatization reaction, advantageously. In the reaction in the embodiment, without using any base reagent but by using, as the base, an amino acid, a peptide, or a catecholamine as the substrate (analyte) containing a nitrogen atom, a derivatization method capable of improving the sensitivity of a nitrogen-containing compound is provided, advantageously.

To the aldehyde or ketone and the derivatization reagent containing the aldehyde or ketone used in the second embodiment, the above explanation for "the aldehyde or ketone" may be appropriately applied. The aldehyde or ketone is preferably a compound represented by Formula (1) (RR'CO). More preferably, R and R' in Formula (1) are each independently a hydrogen atom or a substituted or unsubstituted hydrocarbon group (preferably an alkyl group or an aryl group). R and R' are not specifically limited but are preferably a hydrogen atom or a hydrocarbon group having 1 to 18 carbon atoms, more preferably a hydrogen atom or a hydrocarbon group having 1 to 11 carbon atoms, and even more preferably a hydrogen atom or a hydrocarbon group having 1 to 9 carbon atoms. The alkyl group is preferably a chain (straight chain or branched chain) alkyl group or a cyclic alkyl group, and the aryl group is preferably an unsubstituted aryl group. In a preferred embodiment, (a) when the nitrogen-containing compound is an amino acid or a peptide in a biological substance, R and R' in Formula (1) are a hydrogen atom or a substituted or unsubstituted alkyl group having 1 to 8 carbon atoms. Alternatively, (b) when the nitrogen-containing compound is a catecholamine in a biological substance, R and R' in Formula (1) are a substituted or unsubstituted alkyl group having 2 to 9 carbon atoms.

### 3-1. Reducing agent

The reducing agent used in the present invention is not specifically limited. The reducing agent may be in the liquid form or the solid form or may be dissolved in a solvent or the like into a solution when used.

Examples of the reducing agent include boron compounds (such as a borane, a complex containing a borane, and a borohydride compound salt); aluminum hydrides (such as lithium aluminum hydride, diisobutylaluminum hydride, and sodium bis(2-methoxyethoxy)aluminum hydride); tributyltin hydride; and hydrogen gas. One or two or more kinds selected from them may be used.

Of them, the boron compound is preferred from the viewpoint of easy removal after derivatization.

The boron compound used in the present invention may be any boron compound having reduction activity, and examples include boranes (such as borane (BH₃) and diborane (B₂H₆)), borane-containing complexes (hereinafter also referred to as "borane complexes"), and borohydride compound salts. One or two or more kinds selected from them is preferably used.

The reducing agent used in the present invention may be a commercial product or may be produced with reference to a known production method. The boron compound is advantageously used to easily advance the derivatization of an analyte with the derivatization reagent even at room temperature. Another advantage is that the reducing agent component after the derivatization reaction is easily removed by vacuum concentration with an evaporator or the like.

Examples of the borane complex include borane-2-methylpyridine complex (also referred to as picoline borane), borane-dimethylsulfide (DMS) complex, and borane-amine complexes (such as ammonia borane (or borazane), borane-trimethylamine complex, borane-N,N-diisopropylethylamine complex, and borane-tert-butylamine complex). Of them, borane-2-methylpyridine complex is preferred.

Examples of the borohydride compound salt include alkali metal borohydrides (such as lithium borohydride, sodium borohydride, and potassium borohydride), alkaline earth metal borohydrides (such as calcium borohydride), alkali metal cyanoborohydrides (such as sodium cyanoborohydride), alkali metal triacetoxyborohydrides (such as sodium triacetoxyborohydride), alkali metal triethylborohydrides (such as lithium triethylborohydride), alkali metal tri(sec-butyl)borohydrides (such as lithium tri(sec-butyl)borohydride), and alkaline earth metal tri(sec-butyl)borohydrides (such as potassium tri(sec-butyl)borohydride). Of them, alkali metal borohydrides are preferred.

### 3-2. Acid

The acid used in the present invention is not specifically limited. The acid may be in the liquid form or the solid form or may be dissolved in a solvent or the like into a solution when used.

The acid is preferably a volatile acid from the viewpoint of easy removal after reaction. Examples of the acid include organic acids such as acetic acid, formic acid, propionic acid, lactic acid, and citric acid; inorganic acids such as phosphoric acid, sulfuric acid, and nitric acid; and salts thereof. One or two or more kinds selected from them may be used. Of them, an organic acid is preferred. The acid used in the present invention is preferably an acid (for example, acetic acid) that is easily removed by using an evaporator or the like.

### 3-3. Solvent

The solvent used in the present invention is not specifically limited but is preferably a volatile solvent having a boiling point of 100°C or less. For example, one or two or more kinds solvent selected from water, lower alcohols (for example, alcohols having 1 to 3 carbon atoms, such as methanol, ethanol, n-propanol, and isopropanol), and the like may be used. The solvent may be contained or used in the derivatization reagent or the derivatization reagent kit. The solvent may be used as a solution such as a solution containing an aldehyde or a ketone and a solution containing a reducing agent. By using a solvent that can be removed using an evaporator or the like, a borane reducing agent is removed together.

### 3-4. Amount of component used in the present invention

The concentration of the aldehyde or ketone used in the derivatization reaction is not specifically limited, but is preferably 5 to 100% (v/v) relative to the sample (1 to 100,000 pg).

The concentration of the reducing agent used in the derivatization reaction is not specifically limited, but is preferably 1 to 5 mg/mL relative to the sample (1 to 100,000 pg).

The concentration of the acid used in the derivatization reaction is not specifically limited, but is preferably 8 to 20% (v/v) relative to the sample (1 to 100,000 pg).

### 3-5. Third embodiment pertaining to the present invention (usage of each reagent contained in reagent kit and method for producing derivatized nitrogen-containing compound)

The third embodiment provides a method for derivatizing a nitrogen-containing compound or a method for producing a derivatized nitrogen-containing compound. Each method comprises reacting the nitrogen-containing compound with the derivatization reagent or the derivatization reagent kit. By derivatizing a nitrogen-containing compound with an aldehyde or a ketone, the signal intensity to a detector or the detection sensitivity is improved. As the advantages of the present invention, even when a sample contains a small amount of a nitrogen-containing compound, the analyte in the sample is more accurately detected or determined.

The present invention preferably comprises separating and purifying a nitrogen-containing compound from a sampled sample (preferably a biological substance). The separating and purifying may be carried out as a pretreatment. Accordingly, impurities contained in the sample (preferably the biological substance) are removed, and this enables an improvement in the detection sensitivity or the measurement sensitivity of an analyte.

In the third embodiment, after the reaction, the derivatized nitrogen-containing compound is preferably separated and purified. Accordingly, the unnecessary derivatization reagent after the reaction is removed, and this enables an improvement in the detection sensitivity or the measurement sensitivity of an analyte.

In the third embodiment, the derivatized nitrogen-containing compound is preferably subjected to mass spectrometry.

The nitrogen-containing compound as the analyte is preferably the above-described amino acid, peptide, or catecholamine contained in a biological substance and is preferably one or two or more kinds selected from amino acids such as triiodothyronine, thyroxin, and homocysteine; peptides such as angiotensin I, oxytocin, glucagon, insulin, and C-peptide; and catecholamines such as adrenaline, noradrenaline, and dopamine.

The nitrogen-containing compound is preferably derivatized by reacting the nitrogen-containing compound with the derivatization reagent or the derivatization reagent kit.

In a more preferred embodiment, a sample containing the nitrogen-containing compound, an aldehyde or ketone, and a reducing agent are preferably mixed for derivatization. More preferably, three components, an aldehyde or ketone, a reducing agent, and an acid, are added to a sample containing the nitrogen-containing compound, and the whole is mixed. Alternatively, three components, an aldehyde or ketone, a reducing agent, and an acid, may be added to a sample containing the nitrogen-containing compound, the whole may be mixed, and then a base may be further added. Such a sample and each component may be a solution. For example, a derivatization reagent kit comprising a first solution containing an aldehyde or ketone, a second solution containing a reducing agent, a third solution containing an acid, and the like may be used, or a derivatization reagent kit comprising a first container containing an aldehyde or ketone, a second container containing a reducing agent, a third container containing an acid, and the like may be used. The nitrogen-containing compound may be derivatized by using a derivatization apparatus configured to carry out the method of the invention such as the usage and the production method of the embodiment, with these components, the reagent, or the solution. For the derivatization, no base reagent is preferably used.

The derivatization reaction conditions are not specifically limited. The temperature condition for the derivatization is not specifically limited and is preferably 5 to 50°C and more preferably 10 to 40°C. As the advantages of the derivatization of the present invention, the derivatization reaction proceeds even when the temperature is not set to high, and thus temperature control or any apparatus is not needed. Accordingly, any user easily carries out the reaction. In this case, the derivatization temperature is preferably normal temperatures (for example, 10 to 40°C) and more preferably 20 to 30°C. The pressure condition for the derivatization is not specifically limited, and the derivatization reaction proceeds at normal pressure (standard atmospheric pressure: 1 atm) as the advantages of the technique. In the technique, an analyte can be derivatized at normal temperature and pressure, advantageously. The reaction time for the derivatization is not specifically limited. The lower limit of the reaction time is preferably 0.1 hours or more and more preferably 0.5 hours or 1 hour or more, and the upper limit is preferably 24 hours or less and more preferably 18 hours or less. For the derivatization reaction, an acid (such as acetic acid) is preferably added. For the derivatization reaction, a solvent may be used to dissolve or suspend compounds and the like. As the solvent, an aqueous solvent (such as water and a lower alcohol), an organic solvent, or the like may be appropriately used according to the compound or the derivatization reagent.

The derivatized nitrogen-containing compound produced in the third embodiment is preferably a compound in which an amino group (-NHH) of the nitrogen-containing compound is bonded to an alkyl group or an aryl group as R or R' contained in an aldehyde or ketone as the derivatization reagent into a secondary amine compound or a tertiary amine compound.

### 4. Fourth embodiment pertaining to the present embodiment (mass spectrometric method and sensitivity improvement method in mass spectrometry)

The fourth embodiment provides a mass spectrometric method comprising a derivatization step of derivatizing an analyte with an aldehyde or ketone for derivatization, a derivatization reagent, a derivatization reagent kit, or the like, as described above. The fourth embodiment provides a derivatized compound prepared by derivatization reaction with components such as an aldehyde or ketone. The fourth embodiment preferably comprises, before the derivatization step, a pretreatment step of pretreating a sample by a separation and purification method or the like.

The analyte is preferably the above-described nitrogen-containing compound. The derivatization enables an improvement in measurement sensitivity or detection sensitivity of an analyte in mass spectrometry.

The mass spectrometric method may comprise, after the derivatization step, a mass spectrometry step comprising ionizing a compound to be analyzed by mass spectrometry.

The ionization may be carried out, for example, by electrospray ionization (ESI), atmospheric pressure chemical ionization (APCI), or matrix-assisted laser desorption ionization (MALDI) for more efficient ionization and may be carried out by any ionization other than these ionizations.

By ionizing the analyte derivatized in accordance with the fourth embodiment by such an ionization technique, the measurement sensitivity or the detection sensitivity of the analyte is improved.

The mass spectrometry step may be carried out, for example, by using a liquid chromatography tandem mass spectrometer (LC-MS/MS) or a matrix-assisted laser desorption-ionization mass spectrometer (MALDI-MS), but the apparatus for carrying out the mass spectrometry step is not limited to them.

The specific procedure in the mass spectrometry step may be appropriately designed according to the used ionization method and the used apparatus. The present embodiment includes, for example, a mass spectrometer, a pretreatment apparatus configured to pretreat a sample by a separation and purification method, a derivatization apparatus configured to derivatize a sample, a gas chromatography apparatus, and an HPLC apparatus. One or two or more kinds selected from them may be used.

The fourth embodiment enables the detection or measurement of an N-derivatized analyte and fragment ions thereof (characteristic fragment ions) by mass spectrometry, enables determination of whether the analyte is contained in a sample on the basis of the result, and enables measurements such as detection or quantification of the analyte in a sample.

In the fourth embodiment, to detect or measure an analyte contained in a biological substance in a sample by mass spectrometry, a compound identical to the analyte may be used as a standard, and the result of the analyte may be compared with that of the standard. In other words, by comparing the derivatized compound of an analyte in a biological substance with the derivatized compound of the standard, the analyte in the biological substance is detected or measured.

The method relating to the present embodiment such as the fourth embodiment (for example, the derivatization method, the mass spectrometric method, the measurement method for determining an improvement in sensitivity, or the detection method) may be controlled by a controller comprising a CPU and the like. The method in the fourth embodiment or the like may use, for detection or measurement, One or two or more kinds selected from known methods including a standard addition method, an external standard method (absolute standard method), an internal standard method, an area percentage method, and a corrected area percentage method. The CPU included in the mass spectrometer or in the controller (such as a computer, a server, and a cloud) of the spectrometer may be used to compare data (such as the molecular fragment ion, characteristic fragment ions, a fragment ion pattern, peak heights and areas, and the retention time) of the derivatized compound of an analyte stored in a memory with those of the biological substance in a sample, and measurement including identification, detection, and quantification may be carried out.

5. The present technique may appropriately include different aspects described below or configurations or technical features described below.
[1] A derivatization reagent comprising an aldehyde or a ketone. The derivatization reagent is preferably for mass spectrometry. The aldehyde or the ketone is preferably an aliphatic or aromatic aldehyde or an aliphatic or aromatic ketone.
[2] The derivatization reagent according to the item [1], in which the aldehyde or the ketone is a compound represented by Formula (1):

   RR'CO (1)

   [in Formula (1), R and R' are identical or different, each independently a hydrogen atom or a substituted or unsubstituted hydrocarbon group].
[3] The derivatization reagent according to the item [2], in which the hydrocarbon groups of R and R' in Formula (1) are each independently an alkyl group or an aryl group.
[4] The derivatization reagent according to any one of the items [1] to [3], used to N-alkylate or N-arylate an amino group of a nitrogen-containing compound.
[5] The derivatization reagent according to the item [4], in which the nitrogen-containing compound is at least one selected from amino acids, peptides, and catecholamines contained in a sample (preferably a biological substance).
[6] The derivatization reagent according to any one of the items [1] to [5], in which the derivatization reagent is used to N-alkylate or N-arylate an amino group of a nitrogen-containing compound, and
   (a) when the nitrogen-containing compound is an amino acid or a peptide in a sample (preferably a biological substance), R and R' in Formula (1) are each independently a hydrogen atom or a substituted or unsubstituted alkyl group having 1 to 8 carbon atoms, or
   (b) when the nitrogen-containing compound is a catecholamine in a sample (preferably a biological substance), R and R' in Formula (1) are each independently a substituted or unsubstituted alkyl group having 2 to 9 carbon atoms.
[7] The derivatization reagent according to any one of the items [1] to [6], in which a reducing agent is used for derivatization. More preferably, an acid reagent is further used for derivatization (for example, the reagents are simultaneously or separately added) and/or no base reagent is used for derivatization.
[8] A reducing agent using the derivatization reagent according to any one of the items [1] to [6] for derivatization. More preferably, an acid reagent is further used for derivatization and/or no base reagent is used for derivatization.
[9] A derivatization reagent kit for mass spectrometry, the reagent kit comprising the derivatization reagent according to any one of the items [1] to [7] (a first reagent) and a reducing agent (a second reagent). The reagent kit preferably further comprises an acid (a third reagent). The kit may be a combination or a set. Each reagent of the reagent kit is preferably contained in an openable and closable, airtight container (such as a glass or plastic container or a screw-cap container).
[10] The derivatization reagent kit according to the item [8] or [9], in which the reduction agent comprises at least one selected from a borane, a complex containing a borane, and a salt of a borohydride compound.
[11] The derivatization reagent kit according to any one of the items [8] to [10], further comprising an acid reagent.
[12] The derivatization reagent kit according to any one of the items [8] to [11], in which no base reagent is contained, or no base reagent is used. Preferably, the derivatization reagent kit uses no base reagent for derivatization.
[13] A method for producing a derivatized compound, a method for derivatizing an analyte, or a method for improving the sensitivity of an analyte in mass spectrometry, the method using the derivatization reagent according to any one of the items [1] to [7] or the derivatization reagent kit according to any one of the items [8] to [12] for an analyte.
[14] A method for producing a derivatized compound, a method for derivatizing an analyte, or a method for improving the sensitivity of an analyte in mass spectrometry, the method comprising a derivatization step using the derivatization reagent according to any one of the items [1] to [7] or the derivatization reagent kit according to any one of the items [8] to [12] for an analyte.
[15] The method according to the item [13] or [14] comprising a pretreatment step of separating an analyte from a sample sampled from an animal or the like and the derivatization step.
[16] The method according to any one of the items [13] to [15], comprising the derivatization step and an aftertreatment step of separating the derivatized analyte or removing unnecessary reagents after the reaction.
[17] The method according to any one of the items [13] to [16], comprising an analysis step of analyzing the derivatized analyte by instrumental analysis.
[18] The method according to any one of the items [13] to [17], comprising derivatization of N-alkylating or N-arylating an amino group of a nitrogen-containing compound contained in a sample with a derivatization reagent comprising an aldehyde or a ketone into a secondary amine or a tertiary amine, in which
   the nitrogen-containing compound is at least one selected from amino acids, peptides, and catecholamines contained in a biological substance, and
   the aldehyde or ketone is a compound represented by Formula (1):

      RR'CO (1)

      [in Formula (1), R and R' are a hydrogen atom, a substituted or unsubstituted alkyl group having 1 to 11 carbon atoms, or a substituted or unsubstituted aryl group having 1 to 11 carbon atoms].
[19] An aldehyde or ketone or use thereof for the production of a derivatization reagent or a derivatization reagent kit. More preferably, a reducing agent is used, an acid reagent is used, and/or no base reagent is used.
[20] An aldehyde or ketone or use thereof for the production of a derivatization reagent or a derivatization reagent kit, or an aldehyde or ketone used to produce a derivatization reagent or a derivatization reagent kit. A reducing agent and/or an acid reagent is preferably used, and no base reagent is preferably used.
[21] An aldehyde or ketone for derivatization, or a mixture for derivatization, the mixture comprising an aldehyde or ketone and a reducing agent. More preferably, an acid reagent is contained and/or no base reagent is contained.
[22] An aldehyde or ketone used for a combination product for derivatization, or an aldehyde or ketone and a reducing agent (more preferably an aldehyde or ketone, a reducing agent, and an acid reagent) each used for a derivatization kit product. More preferably, the derivatization kit product comprises no base reagent.
   The kit product may be a combination product or a set product.
[23] The aldehyde or ketone according to the item [22] or the combination product according to the item [20], in which an analyte to be derivatized is a nitrogen-containing compound (preferably a nitrogen-containing compound in a biological substance).
[24] A method for derivatizing a nitrogen-containing compound or a mass spectrometric method of a nitrogen-containing compound, the method using an aldehyde or a ketone selected as a derivatization reagent for improving the sensitivity in mass spectrometry, the aldehyde or ketone being selected by a method of screening a derivatization reagent for improving the sensitivity of a nitrogen-containing compound in mass spectrometry.
[25] A mass spectrometric method using an N-alkylated or N-arylated nitrogen-containing compound prepared by using the derivatization reagent according to any one of the items [1] to [7] or the derivatization reagent kit according to any one of the items [8] to [12].
[26] A method of screening a derivatization reagent for improving the sensitivity of a nitrogen-containing compound in mass spectrometry, the screening method comprising
   a derivatization step by mixing a reagent containing an aldehyde or a ketone with a nitrogen-containing compound, and
   a step of determining a derivatization reagent that improves the sensitivity of a nitrogen-containing compound in mass spectrometry when a nitrogen-containing compound derivatized by the derivatization reagent gives a larger characteristic signal in mass spectrometry than a characteristic signal of the nitrogen-containing compound before derivatization in mass spectrometry. More preferably, in the derivatization step, a reducing agent and/or an acid reagent is used as a reagent, and/or no base reagent is used.
[27] The screening method according to the item [24], wherein the reagent containing an aldehyde or a ketone is the derivatization reagent comprising an aldehyde or a ketone according to any one of the items [1] to [7].
[28] A derivatization reagent kit comprising the aldehyde or ketone or the reagent comprising the aldehyde or ketone determined as the derivatization reagent for improving the sensitivity in the item [26] or [27].

### Examples

The present technique will next be described in further detail with reference to examples and the like. The examples and the like described below are merely typical examples and the like of the present technique, and the scope of the technique is not intended to be limited to them.

### [Test Example 1: Production of a derivatized compound as a secondary amine or a tertiary amine by reacting a standard (an analyte) with a derivatization reagent (aldehyde or ketone reagent)

### 1. Reagents

A group of aldehydes and a ketone shown in Table 1 was used for derivatization.

**[Table 1]**

| Table 1: Reagents (aldehyde: RCHO, ketone: RR'-CO) | |
|---|---|
| | Substituent after reduction |
| Formaldehyde (HCHO) | Me |
| Acetaldehyde (CH₃CHO) | Et |
| Propionaldehyde (CH₃CH₂CHO) | Pr |
| Cyclopropanecarboxaldehyde (CpCHO) | CpM |
| Butyraldehyde (CH₃CH₂CH₂CHO) | Bu |
| Isobutyraldehyde (CH₃(CH₃)CHCHO) | IB |
| Valeraldehyde (CH₃CH₂CH₂CH₂CHO) | V |
| Isovaleraldehyde (CH₃(CH₃) CH₂CH₂CHO) | IV |
| Hexanal (CH₃CH₂CH₂CH₂CH₂CHO) | Hx |
| Heptanal (CH₃CH₂CH₂CH₂CH₂CH₂CHO) | Hp |
| Benzaldehyde (PhCHO) | Bn |
| Acetone (CH₃COCH₃) | iPr |

| | |
|---|---|
| · An aqueous 5% aldehyde or ketone solution (a 10% aqueous solution for formaldehyde, a 5% to 10% aqueous ethanol solution for hexanal, heptanal, and benzaldehyde, a 50% or 100% aqueous solution for acetone) · A 3 mg/mL borane-2-picoline complex solution in isopropanol (preparation as needed) · Acetic acid | |

### 2. Derivatization

### [Standard (analyte)]

· Amino acids (triiodothyronine (T3) having a molecular weight of 651.0 and a hydrophobicity (ClogP) of 2.63, thyroxin (T4) having a molecular weight of 776.9 and a hydrophobicity (ClogP) of 3.51, homocysteine (HCys) having a molecular weight of 135.2 and a hydrophobicity (ClogP) of -2.07)
· Peptides (angiotensin I (AI) having a molecular weight of 1296.5 and a hydrophobicity (ClogP) of -2.70, oxytocin (OXT) having a molecular weight of 1007.2 and a hydrophobicity (ClogP) of -0.65, glucagon (GN) having a molecular weight of 3482.8 and an unknown hydrophobicity (ClogP), adrenocorticotropic hormone (ACTH) having a molecular weight of 4579.3 and an unknown hydrophobicity (ClogP), insulin (Ins) having a molecular weight of 5807.6 and an unknown hydrophobicity (ClogP), C-peptide (CP) having a molecular weight of 3020.3 and an unknown hydrophobicity (ClogP))
· Catecholamines (adrenaline (Ad) having a molecular weight of 183.2 and a hydrophobicity (ClogP) of -0.68, noradrenaline (NA) having a molecular weight of 169.2 and a hydrophobicity (ClogP) of -0.99, dopamine (DP) having a molecular weight of 153.2 and a hydrophobicity (ClogP) of 0.17)

### 3. Analysis conditions

### [LC analysis conditions]

Apparatus: Shimadzu LC-20A
Analytical column: YMC Triart C18 (2.1 × 50 mm)
Elution conditions: flow rate, 0.4 mL/min; solvent A, 0.1% formic acid-water; solvent B, methanol
Time (min) [solvent B%]: 0 min [0%], 3 min [0%], 3.5 min [100%], 4 min [0%], 5 min [0%]

### [MS/MS analysis conditions]

Apparatus: Shimadzu LCMS8040 triple-quadrupole mass spectrometer
Ionization conditions: ESI positive ion mode
Collision energy (CE) and selected reaction monitoring (SRM) chromatograms are shown in Table below.

### [MS analysis]

Apparatus: Shimadzu LCMS8040 triple-quadrupole mass spectrometer
Ionization conditions: ESI positive ion mode
Measurement was carried out in a scan mode, and the mass spectrum was shown. The spectrum horizontal axis was m/Z.

### [¹H-NMR analysis]

Apparatus: JEOL JNM-ECA500 nuclear magnetic resonance spectrometer
A standard (0.1 mg to 1.0 mg) was derivatized, and the product was measured 1,024 scans (16 scans for HCys) at room temperature (22°C) to give a spectrum. The spectrum horizontal axis was ppm.

### [Derivatization]

To a standard (1 pg to 100 ng), 25 µL of an aqueous aldehyde or ketone solution, 25 µL of an aqueous picoline borane solution, and 5 µL of acetic acid were added, and the mixture was allowed to stand at room temperature (20 to 30°C) for 30 minutes for derivatization. After the derivatization, the aldehyde or ketone, the acetic acid, the boron, and the like were removed by concentration with an evaporator. After the concentration with the evaporator, the residue was dissolved in a redissolving solvent (an aqueous 30% acetonitrile solution), and the solution was subjected to LC-MS/MS analysis.

### 4. Derivatization result of amino acid standard

A standard (analyte: an amino acid present in a biological substance) was reacted with an aldehyde or a ketone to give a secondary amine or a tertiary amine as a derivatized compound.

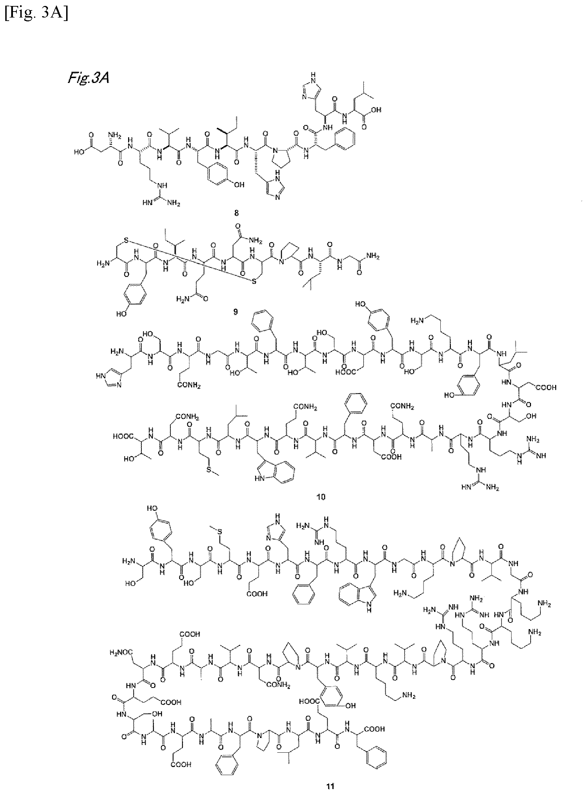

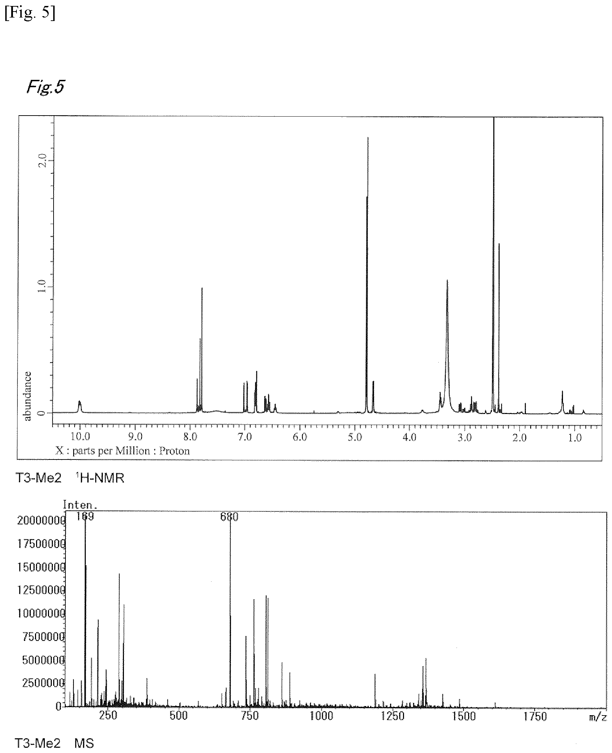

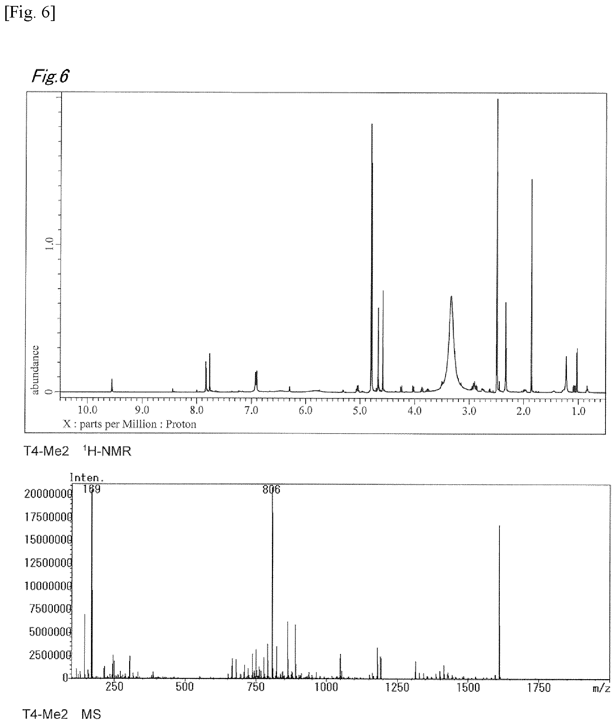

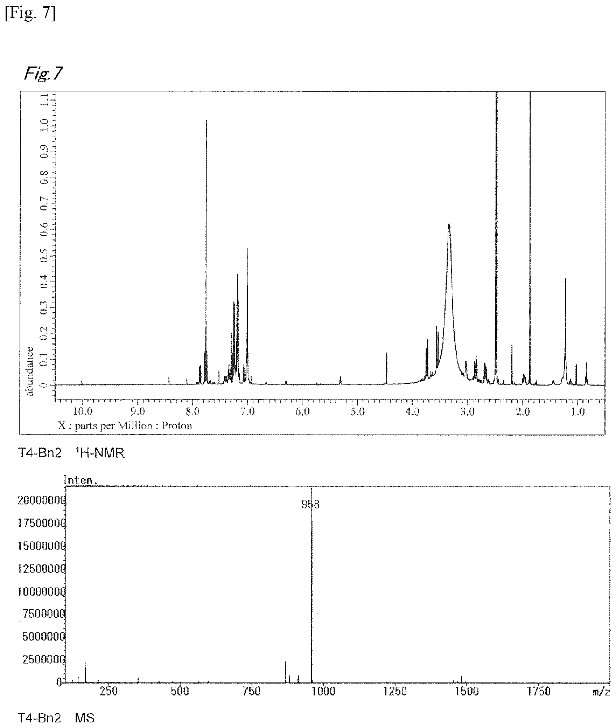

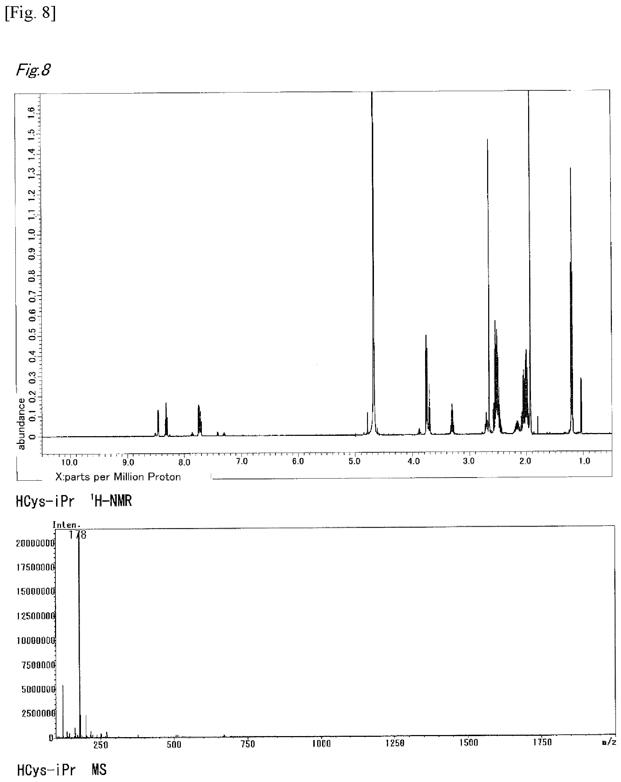

Specifically, triiodothyronine (T3) and thyroxin (T4) were each reacted with formaldehyde. Thyroxin (T4) as a standard (analyte) was reacted with benzaldehyde. Each gave a secondary amine or a tertiary amine as the derivatized compound. Homocysteine (HCys) was reacted with acetone. This gave a secondary amine as the derivatized compound. These derivatized compounds were identified by ¹H-NMR analysis and MS analysis to be T3-Me2 (N,N-dimethyltriiodothyronine), T4-Me2 (N,N-dimethylthyroxin), and T4-Bn2 (N,N-dibenzylthyroxin), and HCys-iPr (N-isopropylhomocysteine) (Fig. 1 to Fig. 8). Accordingly, it was ascertained that amino acids (specifically amino acids present in a biological substance) were able to be derivatized with an aldehyde or a ketone having 1 to 8 carbon atoms.

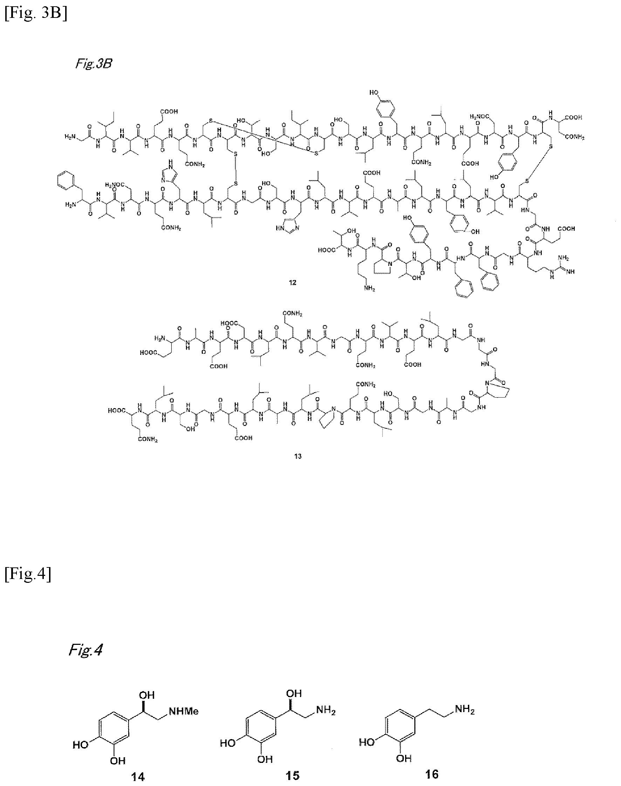

SRM parameters for the standards (not derivatized) and compounds derivatized with an aldehyde or a ketone are shown in Tables 2 to 4 (no SRM chromatogram (10 ng) is shown). Table 5 shows the results of the derivatized compounds in which the relative sensitivity improvement ratio was high and the detection sensitivity or the measurement sensitivity was greatly improved. ¹H-NMR and MS spectra thereof are shown in Figs. 4 to 8.

As shown by the results, the sensitivity of the derivatized compounds of amino acids (specifically amino acids present in a biological substance) with an aldehyde or ketone (specifically an aldehyde or ketone having 1 to 8 carbon atoms) was higher than the sensitivity of the standards. By using the aldehyde or ketone as the derivatization reagent, the detection sensitivity was improved.

**[Table 2]**

| Table 2: SRM parameters for triiodothyronine (T3) | | |
|---|---|---|
| Parent ion (M+H⁺) | m/z | CE(V) |
| T3 (not derivatized) | 651.9>605.8 | 22 |
| T3-Me₂ | 679.8>633.7 | 32 |
| T3-Et | 679.8>633.7 | 29 |
| T3-Pr | 693.8>647.7 | 30 |
| T3-CpM | 705.9>605.6 | 27 |
| T3-Bu | 707.8>661.8 | 35 |
| T3-IB | 707.9>661.7 | 29 |
| T3-V₂ | 791.9>43.1 | 55 |
| T3-IV₂ | 791.9>43.1 | 55 |
| T3-Hx₂ | 820.1>689.7 | 39 |
| T3-Hp₂ | 848.1>801.9 | 29 |
| T3-Bn | 741.9>91.0 | 39 |
| T3-iPr | 693.9>605.65 | 33 |

**[Table 3]**

| Table 3: SRM parameters for thyroxin (T4) | | |
|---|---|---|
| Parent ion (M+H⁺) | m/z | CE(V) |
| T4 (not derivatized) | 777.8>731.6 | 30 |
| T4-Me₂ | 805.8>759.6 | 36 |
| T4-Et₂ | 833.7>787.6 | 41 |
| T4-Pr₂ | 861.75>815.7 | 39 |
| T4-CpM | 831.8>731.5 | 35 |
| T4-Bu₂ | 889.9>843.8 | 39 |
| T4-IB | 833.8>787.7 | 42 |
| T4-V₂ | 917.8>801.7 | 46 |
| T4-IV₂ | 917.8>43.1 | 55 |
| T4-Hx₂ | 946>815.5 | 45 |
| T4-Hp | 875.9>829.7 | 41 |
| T4-Bn₂ | 957.7>91.0 | 54 |
| T4-iPr | 819.6>731.5 | 40 |

**[Table 4]**

| Table 4: SRM parameters for homocysteine (HCys)* | | |
|---|---|---|
| Parent ion (M+H⁺) | m/z | CE(V) |
| HCys (not derivatized) | 136.1>89.9 | 14 |
| HCys-Me₂ | 164.2>45.0 | 16 |
| HCys-Et₂ | 192.1>119.0 | 18 |
| HCys-Pr | 178.2>122.1 | 15 |
| HCys-CpM | 190.3>158.1 | 9 |
| HCys-Bu | 192.1>119.1 | 16 |
| HCys-IB | 192.3>119.0 | 18 |
| HCys-V₂ | ND | ND |
| HCys-IV₂ | ND | ND |
| HCys-Hx₂ | ND | ND |
| HCys-Hp₂ | 332.2>46.1 | 10 |
| HCys-Bn₂ | NO | ND |
| HCys-iPr | 178.1>90.0 | 16 |

| | | |
|---|---|---|
| *ND: not determined | | |

**[Table 5]**

| Table 5: Relative sensitivity improvement of standards (amino acids)* | | | |
|---|---|---|---|
| N-alkyl or aryl group | Me | Bn | iPr |
| T3 (10 pg) | 1.4 | - | - |
| T4 (10 pg) | 2.5 | 1.6 | - |
| HCys (10 ng) | - | - | 2.1 |

| | | | |
|---|---|---|---|
| * Relative sensitivity improvement ratios where the sensitivity of the amino acid not derivatized is 1. | | | |

### 5. Derivatization result of peptide standard

A standard (analyte: a peptide present in a biological substance) was reacted with an aldehyde or a ketone to give a secondary amine or a tertiary amine as a derivatized compound.

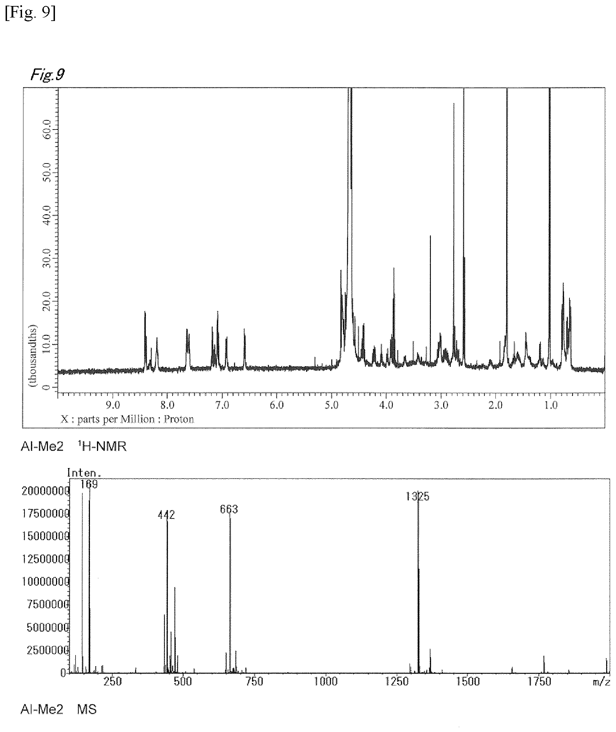

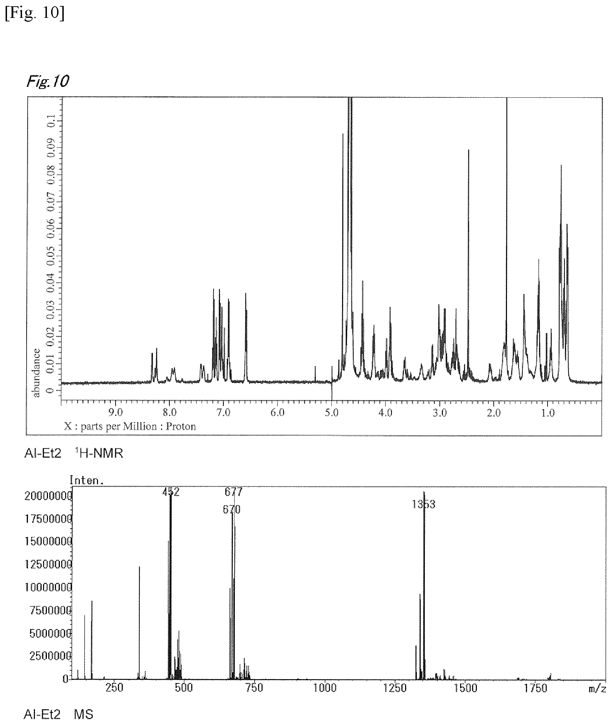

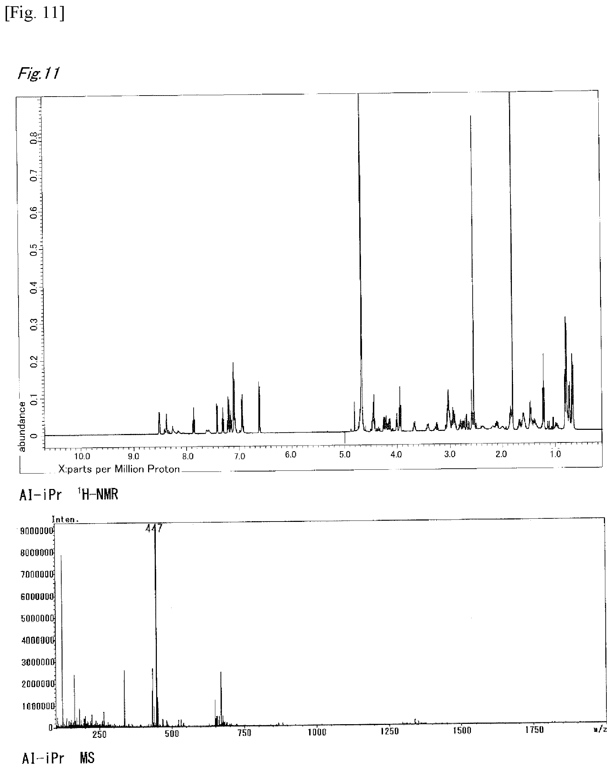

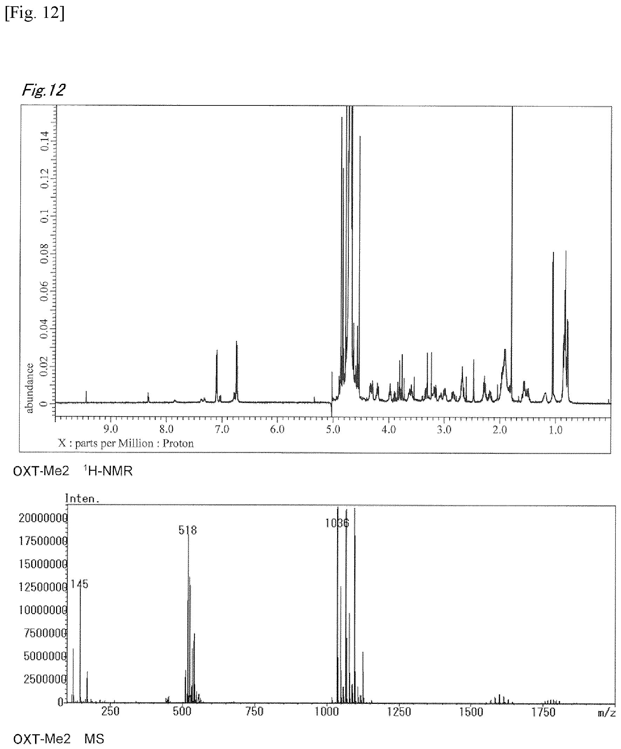

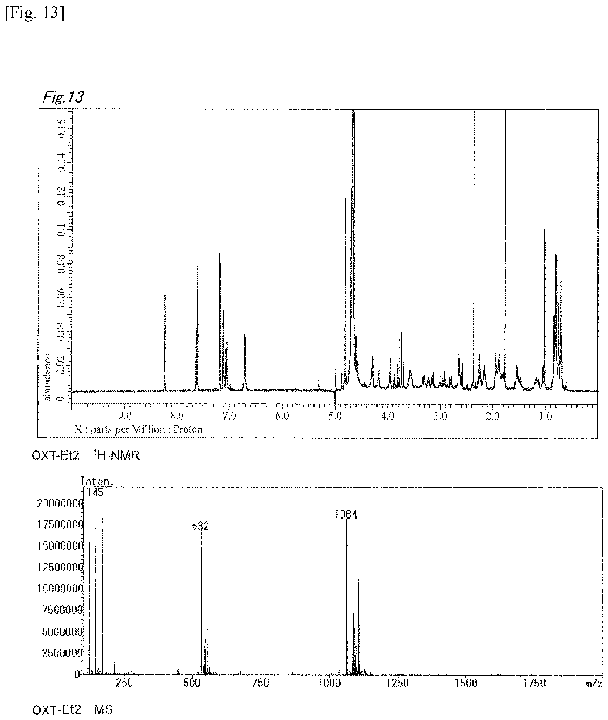

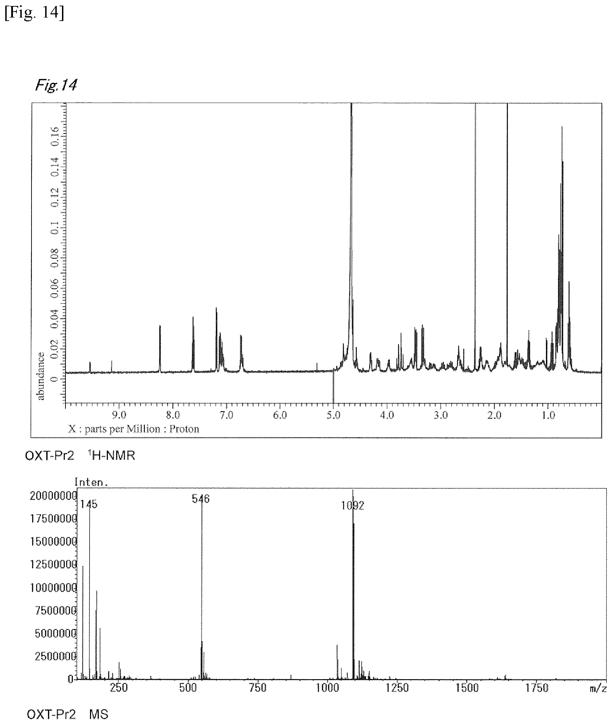

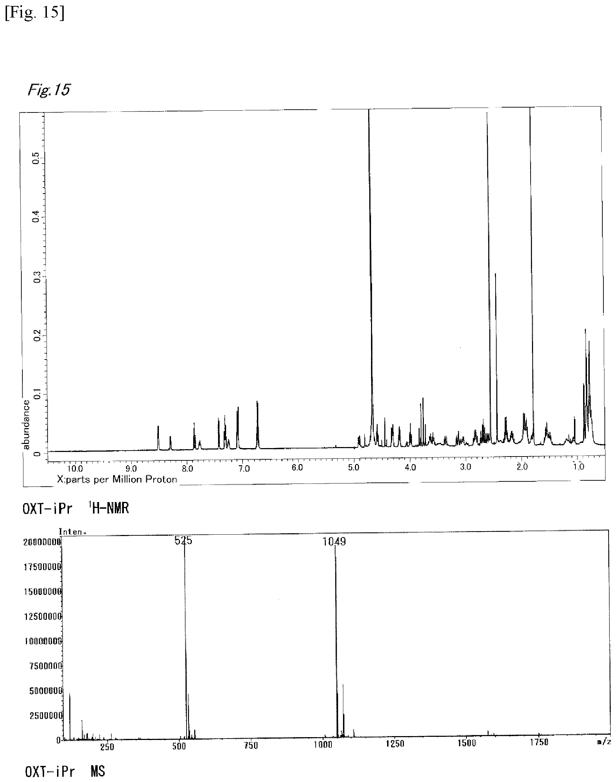

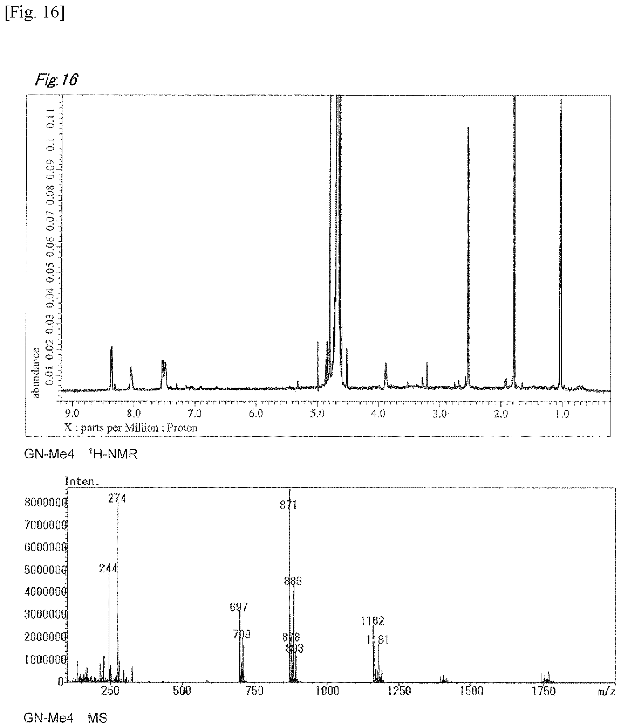

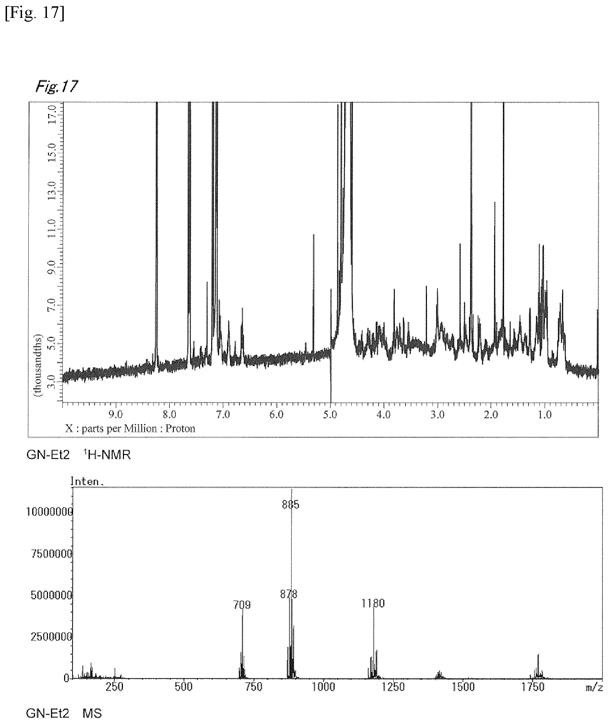

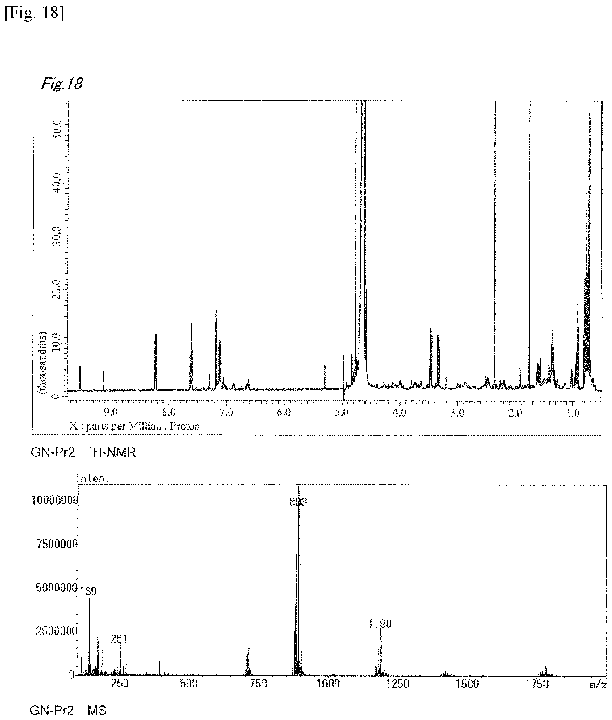

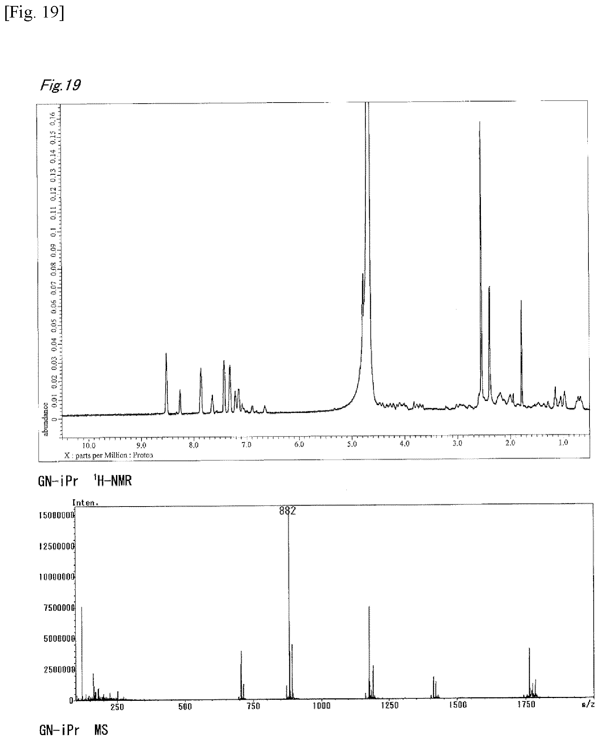

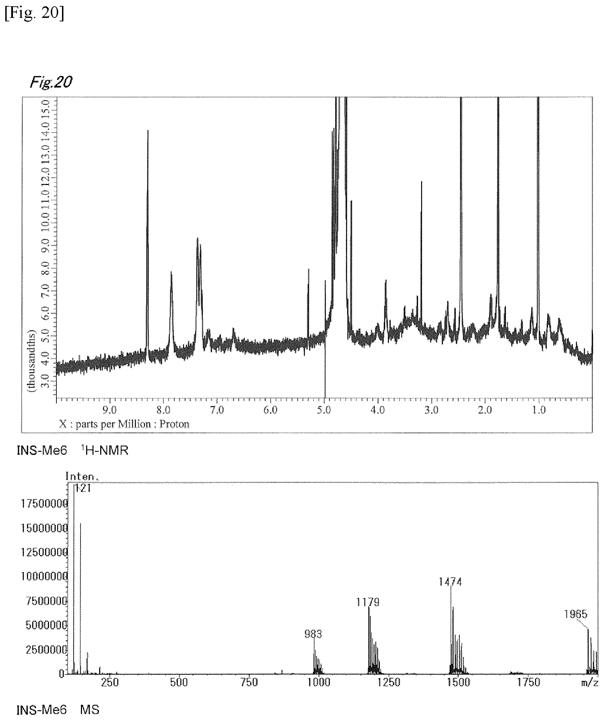

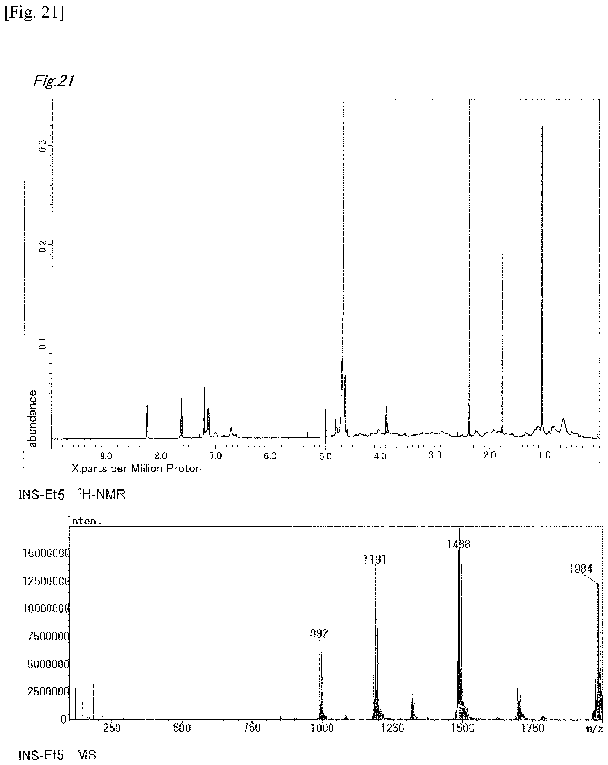

Specifically, angiotensin I (AI) was reacted with formaldehyde, acetaldehyde, or acetone. Oxytocin (OXT) was reacted with formaldehyde, acetaldehyde, propionaldehyde, or acetone. Glucagon (GN) was reacted with formaldehyde, acetaldehyde, propionaldehyde, or acetone. Insulin (INS) was reacted with formaldehyde or acetaldehyde. Each standard gave a secondary amine or a tertiary amine as the derivatized compound. The derivatized compounds of AI were identified by ¹H-NMR analysis and MS analysis to be AI-Me2 (N,N-dimethylangiotensin I), AI-Et2 (N,N-diethylangiotensin I), and AI-iPr (N-isopropylangiotensin I) (Figs. 9 to 11). The derivatized compounds of OXT were identified by ¹H-NMR analysis and MS analysis to be OXT-Me2 (N,N-dimethyloxytocin), OXT-Et2 (N,N-diethyloxytocin), OXT-Pr2 (N,N-dipropyloxytocin), and OXT-iPr (N-isopropyloxytocin) (Figs. 12 to 15). The derivatized compounds of GN were identified to be GN-Me4 (N,N,N',N'-tetramethylglucagon), GN-Et2 (N,N'-diethylglucagon), GN-Pr2 (N,N'-dipropylglucagon), and GN-iPr (N-isopropylglucagon) (Figs. 16 to 19). The derivatized compounds of INS were identified by ¹H-NMR analysis and MS analysis to be INS-Me6 (N,N,N',N',N",N"-hexamethylinsulin) and INS-Et5 (N,N,N',N',N"-pentaethylinsulin). Accordingly, it was ascertained that peptides (specifically peptides present in a biological substance) were able to be derivatized with an aldehyde having 1 to 8 carbon atoms (Figs. 20 and 21).

SRM parameters for the standards (not derivatized) and compounds derivatized with an aldehyde or a ketone are shown in Tables 6 to 11 (SRM chromatogram (10 ng) not shown). Table 12 shows the result of derivatized compounds giving a high relative sensitivity improvement ratio, and the identification results of them are shown in Figs. 9 to 21.

As shown by the results, the sensitivity of the derivatized compounds of peptides (specifically peptides present in a biological substance) with an aldehyde or a ketone (specifically an aldehyde or ketone having 1 to 8 carbon atoms) was higher than the sensitivity of the standard. By using the aldehyde or ketone as the derivatization reagent, the detection sensitivity was improved.

**[Table 6]**

| Table 6: SRM parameters for angiotensin I (Al)* | | |
|---|---|---|
| Parent ion (M+3H⁺) | m/z | CE(V) |
| AI (not derivatized) | 433>110.0 | 31 |
| AI-Me₂ | 442.3>110.0 | 40 |
| AI-Et₂ | 451.7>110.1 | 36 |
| AI-Pr | 447>110.1 | 35 |
| AI-CpM | ND | ND |
| AI-Bu | 451.7>110.1 | 30 |
| AI-IB | 451.7>110.1 | 29 |
| AI-V₂ | 479.7>110.1 | 35 |
| AI-IV₂ | 479.7>110.1 | 35 |
| AI-Hx | 461.1>110.1 | 33 |
| AI-Hp | 465.7>110.0 | 35 |
| AI-Bn | 463.1 >110.1 | 35 |
| AI-iPr | 447.1>110.1 | 30 |

| | | |
|---|---|---|
| *ND: not determined | | |

**[Table 7]**

| Table 7: SRM parameters for oxytocin (OXT) | | |
|---|---|---|
| Parent ion (M+H⁺) | m/z | CE(V) |
| OXT (not derivatized) | 1007.5>723.3 | 40 |
| OXT-Me₂ | 1035.6>751.3 | 35 |
| OXT-Et₂ | 1063.5>130.1 | 45 |
| OXT-Pr₂ | 1091.5>158.1 | 55 |
| OXT-CpM | 1075.5>141.9 | 49 |
| OXT-Bu₂ | 1119.55>186.1 | 55 |
| OXT-IB₂ | 1119.55>186.0 | 54 |
| OXT-V₂ | 1147.5>214.0 | 55 |
| OXT-IV₂ | 1147.5>214.1 | 55 |
| OXT-Hx | 1091.5>807.2 | 38 |
| OXT-Hp | 1105.5>821.2 | 38 |
| OXT-Bn | 1097.5>813.1 | 45 |
| OXT-iPr | 1049.5>765.1 | 40 |

**[Table 8]**

| Table 8: SRM parameters for glucagon (GN) | | |
|---|---|---|
| Parent ion (M+4H⁺) | m/z | CE(V) |
| GN (not derivatized) | 871.5>110.0 | 55 |
| GN-Me₄ | 885.8>138.2 | 55 |
| GN-Et₂ | 885.5>166.1 | 50 |
| GN-Pr₂ | 892.5>194.1 | 55 |
| GN-CpM₂ | 898.5>218.0 | 54 |
| GN-Bu₂ | 899.4>222.3 | 55 |
| GN-IB₂ | 899.4>222.2 | 50 |
| GN-V₂ | 906.7>250.3 | 55 |
| GN-IV₂ | 906.7>250.3 | 55 |
| GN-Hx | 892.6>194.2 | 50 |
| GN-Hp | 895.9>208.1 | 54 |
| GN-Bn₂ | 916.5>875.3 | 15 |
| GN-iPr | 882.1>152.0 | 55 |

**[Table 9]**

| Table 9: SRM parameters for adrenocorticotropic hormone (ACTH) | | |
|---|---|---|
| Parent ion (M+5H⁺) | m/z | CE(V) |
| ACTH (not derivatized) | 909.0>120.1 | 44 |
| ACTH-Me₆ | 926.1>119.9 | 27 |
| ACTH-Et₄ | 931.5>120.0 | 50 |
| ACTH-Pr₄ | 942.8>120.0 | 45 |
| ACTH-CpM₂ | 931.5>120.0 | 45 |
| ACTH-Bu₄ | 953.9>120.1 | 55 |
| ACTH-IB₄ | 953.8>120.1 | 45 |
| ACTH-V₄ | 965.1>120.0 | 50 |
| ACTH-IV₄ | 965.1>70.1 | 50 |
| ACTH-Hx₄ | 976.4>120.0 | 55 |
| ACTH-Hp₃ | 968.0>120.0 | 50 |
| ACTH-Bn₃ | 926.5>70.1 | 43 |

**[Table 10]**

| Table 10: SRM parameters for insulin (INS) | | |
|---|---|---|
| Parent ion (M+5H⁺) | m/z | CE(V) |
| INS (not derivatized) | 1162.2>143.0 | 71 |
| INS-Me₆ | 1179.3>148.0 | 64 |
| INS-Et₅ | 1190.5>176.0 | 70 |
| INS-Pr₅ | 1204.5>204.0 | 72 |
| INS-CpM₂ | 1184.0>197.1 | 54 |
| INS-Bu₅ | 1218.6>232.2 | 71 |
| INS-IB₂ | 1184.9>176.0 | 75 |
| INS-V₄ | 1218.6>260.2 | 65 |
| INS-IV₄ | 1218.6>260.1 | 70 |
| INS-Hx₂ | 1196.1>204.2 | 65 |
| INS-Hp₂ | 1201.6>136.2 | 55 |
| INS-Bn₂ | 1198.5> 136.2 | 49 |

**[Table 11]**

| Table 11: SRM parameters for C-peptide (CP) | | |
|---|---|---|
| Parent ion (M+3H⁺) | m/z | CE(V) |
| CP (not derivatized) | 1007.5>147.1 | 34 |
| CP-Me₂ | 1017>147.1 | 38 |
| CP-Et | 1017>147.0 | 30 |
| CP-Pr | 1021.6>147.0 | 35 |
| CP-CpM | 1025.5>146.9 | 47 |
| CP-Bu | 1026.3>147.0 | 35 |
| CP-IB | 1026.3>147.0 | 45 |
| CP-V | 1031>147.0 | 44 |
| CP-IV | 1030.9>147.1 | 35 |
| CP-Hx | 1035.6>147.1 | 45 |
| CP-Hp | 1040.3>147.0 | 38 |
| CP-Bn | 1037.6>147.2 | 45 |

**[Table 12]**

| Table 12: Relative sensitivity improvement of standard (peptide)* | | | | |
|---|---|---|---|---|
| N-alkyl group | Me | Et | Pr | iPr |
| Al (10 pg) | 1.1 | 1.7 | - | 1.7** |
| GN (1 ng) | - | 6.2 | 2.4 | 7.1** |
| Ins (100 pg) | 4.9 | 1.4 | - | - |

| | | | | |
|---|---|---|---|---|
| * Relative sensitivity improvement ratios where the sensitivity of the peptide not derivatized is 1. **10 ng | | | | |

### 6. Derivatization result of catecholamine standard

A standard (analyte: a catecholamine present in a biological substance) was reacted with an aldehyde to give a secondary or tertiary amine as a derivatized compound. SRM parameters for the standards (not derivatized) and compounds derivatized with an aldehyde are shown in Tables 13 to 15 (no SRM chromatogram (10 ng) is shown). Specifically, the adrenaline standard yielded Ad-Me, Ad-Et, Ad-Pr, Ad-CpM, Ad-Bu, Ad-IB, Ad-V, Ad-IV, Ad-Hx, Ad-Hp, and Ad-Bn. The noradrenaline standard yielded NA-Me2, NA-Et2, NA-Pr2, NA-CpM, NA-Bu2, NA-IB2, NA-V2, NA-IV2, NA-Hx2, NA-Hp2, and NA-Bn2. The dopamine standard yielded DP-Me2, DP-Et2, DP-Pr2, DP-CpM, DP-Bu2, DP-IB2, DP-V2, DP-IV, DP-Hx, DP-Hp2, and DP-Bn2.

Table 16 shows the result of derivatized compounds giving a high relative sensitivity improvement ratio. As shown by the results, the sensitivity of the derivatized compounds of catecholamines (specifically catecholamines present in a biological substance) with an aldehyde (specifically an aldehyde having 3 to 9 carbon atoms) was higher than the sensitivity of the standard. By using the aldehyde as the derivatization reagent, the detection sensitivity was improved.

**[Table 13]**

| Table 13: SRM parameters for adrenaline (Ad) | | |
|---|---|---|
| Parent ion (M+H⁺) | m/z | CE(V) |
| Ad (not derivatized) | 184.2>166.1 | 11 |
| Ad-Me | 198.0>78.2 | 10 |
| Ad-Et | 212.1>195.1 | 8 |
| Ad-Pr | 226.1>208.2 | 14 |
| Ad-CpM | 238.1>220.1 | 12 |
| Ad-Bu | 240.1>222.1 | 15 |
| Ad-IB | 240.1>222.1 | 14 |
| Ad-V | 254.3>236.2 | 14 |
| Ad-IV | 254.3>236.2 | 13 |
| Ad-Hx | 268.2>250.2 | 15 |
| Ad-Hp | 282.3>264.2 | 15 |
| Ad-Bn | 274.1>256.2 | 13 |

**[Table 14]**

| Table 14: SRM parameters for noradrenaline (NA)* | | |
|---|---|---|
| Parent ion (M+H⁺) | m/z | CE(V) |
| NA (not derivatized) | 170.0>152.2 | 11 |
| NA-Me₂ | 198.0>78.1 | 10 |
| NA-Et₂ | ND | ND |
| NA-Pr₂ | 254.1>236.0 | 15 |
| NA-CpM | 224.1>206.5 | 9 |
| NA-Bu₂ | 282.2>264.3 | 15 |
| NA-IB₂ | 282.2>264.1 | 16 |
| NA-V₂ | 310.2>292.2 | 18 |
| NA-IV₂ | 310.2>292.2 | 17 |
| NA-Hx₂ | 338.2>320.3 | 20 |
| NA-Hp₂ | 366.4>348.3 | 20 |
| NA-Bn₂ | 349.9>267.4 | 7 |

| | | |
|---|---|---|
| *ND: not determined | | |

**[Table 15]**

| Table 15: SRM parameters for dopamine (DP) | | |
|---|---|---|
| Parent ion (M+H⁺) | m/z | CE(V) |
| DP (not derivatized) | 154.0>137.0 | 15 |
| DP-Me₂ | 182.2>135.2 | 12 |
| DP-Et₂ | 210.1>193.2 | 8 |
| DP-Pr₂ | 237.5>219.0 | 8 |
| DP-CpM | 208.1>137.1 | 15 |
| DP-Bu₂ | 266.2>249.3 | 8 |
| DP-IB₂ | 266.2>249.2 | 8 |
| DP-V₂ | 294.2>137.0 | 23 |
| DP-IV | 224.2>207.1 | 8 |
| DP-Hx | 238.34>137.1 | 18 |
| DP-Hp₂ | 350.3>137.1 | 30 |
| DP-Bn₂ | 334.0>91.0 | 33 |

**[Table 16]**

| Table 16: Relative sensitivity improvement of standard (catecholamine)* | | | | | | | | |
|---|---|---|---|---|---|---|---|---|
| N-alkyl group | Pr | CpM | Bu | IB | V | IV | Hx | Hp |
| Ad (1 ng) | 2.4 | 1.2 | 5.4 | 1.8 | 10 | 6.8 | 13 | 14 |
| NA (1 ng) | 8.5 | 2.5 | 44 | 3.5 | 40 | 16 | 7.2 | 93 |
| DP (1 ng) | - | 1.6 | 7.1 | 6.5 | 14 | 1.5 | 7.0 | 35 |

| | | | | | | | | |
|---|---|---|---|---|---|---|---|---|
| * Relative sensitivity improvement ratios where the sensitivity of the catecholamine not derivatized is 1 | | | | | | | | |

### [Test Example 2: Detection of oxytocin in the presence of plasma extract]

### 1. Reagents

· An aqueous 5% aldehyde solution (propionaldehyde, butyraldehyde, valeraldehyde)
· A 3 mg/mL borane-2-picoline complex solution in isopropanol (preparation as needed)
· Acetic acid

### 2. Preparation of plasma extract

A weak cation-exchange column (Waters, Oasis^{(R)} WCX 3 cc, Cartridge 60 mg, 30 µm) was washed with methanol (2 mL) and was equilibrated with water (2 mL). A 4% phosphate buffer (300 µL) and pooled plasma (300 µL) were mixed, and the mixture was loaded onto the column. The column was successively washed with a 5% aqueous ammonia (2 mL) and an aqueous 75% acetonitrile solution (2 mL) and was eluted with a 1% formic acid and 75% acetonitrile solution in water (2 mL). The eluate was concentrated with a centrifugal evaporator to give a plasma extract.

### 3. Derivatization

To the plasma extract, 0, 1, or 10 pg of an oxytocin standard was added, and 25 µL of an aqueous aldehyde solution, 25 µL of a picoline borane solution, and 5 µL of acetic acid were added. The mixture was allowed to stand at room temperature for 30 minutes. After the derivatization, the aldehyde, the acetic acid, the boron, and the like were removed by concentration with an evaporator. After the concentration with the evaporator, the residue was dissolved in a redissolving solvent (a 30% acetonitrile solution), and the solution was subjected to LC-MS/MS analysis.

### 4. LC analysis conditions

Apparatus: Shimadzu LC-20A
Analytical column: YMC Triart C18 2.1 × 50 mm
Elution conditions: flow rate, 0.4 mL/min; solvent A, 0.1% formic acid-water; solvent B, methanol
Time (min) [solvent B%]: 0 min [0%], 3 min [0%], 3.5 min [100%], 4 min [0%], 5 min [0%]

### 5. MS/MS analysis conditions

Apparatus: Shimadzu LCMS8040 triple-quadrupole mass spectrometer
Ionization conditions: ESI positive ion mode
Collision energy (CE) and selected reaction monitoring (SRM) chromatograms are shown in Tables below.

6. Measurement results
The sensitivity improvement effects by N-alkylation are shown in Table 17 and Table 18. The sensitivity improvement of the dipropylated product (OXT-Pr₂) was 3.7 times, that of the dibutylated product (OXT-Bu₂) was 2.4 times, and that of the divalated product (OXT-V₂) was 1.7 times. Hence, N-alkylation improved the detection sensitivity of the peptide in serum or plasma.

**[Table 17]**

| Table 17: SRM parameters for oxytocin (OXT) | | |
|---|---|---|
| Parent ion (M+H⁺) | m/z | CE(V) |
| OXT (not derivatized) | 1007.5>723.3 | 40 |
| OXT-Pr₂ | 1091.5>158.1 | 55 |
| OXT-Bu₂ | 1119.55>186.1 | 55 |
| OXT-V₂ | 1147.5>214.0 | 55 |

**[Table 18]**

| Table 18: Relative sensitivity improvement of oxytocin in the presence of plasma extract | | | | |
|---|---|---|---|---|
| m/z | 1007.5>723.3 | 1091.5>158.1 | 1119.55>186.1 | 1147.5>214.0 |
| *t*_{R}* | 2.816 | 3.024 | 3.065 | 3.162 |
| OXT | 368 | | | |
| OXT-Pr₂ | | 1369 | | |
| OXT-Bu₂ | | | 877 | |
| OXT-V₂ | | | | 623 |
| Sensitivity improvement* * | 1 | 3.7 | 2.4 | 1.7 |

| | | | | |
|---|---|---|---|---|
| **t*_{R}: retention time (min) ** Relative sensitivity improvement ratios where the sensitivity of the peptide not derivatized is 1. | | | | |

The numeric characters in Table are peak areas on SRM chromatograms.

### [Test Example 3: Optimization of derivatization reaction]

The catecholamine derivatization reaction was optimized using a standard (the concentration of an aldehyde reagent, with acetic acid).

### 1. Reagents

· A 10% heptanal-80% ethanol solution in water
· A 3 mg/mL borane-2-picoline complex solution in isopropanol (preparation as needed)
· Acetic acid

### 2. Derivatization

To a catecholamine (adrenaline, noradrenaline, or dopamine) standard (1ng), 25 µL of an aqueous aldehyde solution, 25 µL of a picoline borane solution, and 5 µL of acetic acid were added, and the mixture was allowed to stand at room temperature for 30 minutes. After the derivatization, the aldehyde, the acetic acid, the boron, and the like were removed by concentration with an evaporator. After the concentration , the residue was dissolved in a redissolving solvent (a 30% acetonitrile solution), and the solution was subjected to LC-MS/MS analysis.

### 3. Analysis conditions

LC analysis and MS/MS analysis were carried out under substantially the same analysis conditions as those for the LC analysis and the MS/MS analysis in Test Example 1.

### 4. Results

The sensitivity improvement effects by N-alkylation and arylation are shown in Table 19 and Table 20.

For catecholamines as the analyte, using heptanal as the derivatization reagent improved the sensitivity. Hence, the N-alkylation improved the detection sensitivity of catecholamines.

**[Table 19]**

| Table 19: SRM parameters for catecholamines | | |
|---|---|---|
| SRM parameters for adrenaline (Ad) | | |
| Parent ion (M+H⁺) | m/z | CE(V) |
| Ad (not derivatized) | 184.2>166.1 | 11 |
| Ad-Hp | 282.3>264.2 | 15 |

| SRM parameters for noradrenaline (NA) | | |
|---|---|---|
| Parent ion (M+H⁺) | m/z | CE(V) |
| NA (not derivatized) | 170.0>152.2 | 11 |
| NA-Hp₂ | 366.4>348.3 | 20 |

| SRM parameters for dopamine (DP) | | |
|---|---|---|
| Parent ion (M+H⁺) | m/z | CE(V) |
| DP (not derivatized) | 154.0>137.0 | 15 |
| DP-Hp₂ | 350.3>137.1 | 30 |

**[Table 20]**

| Table 20: Relative sensitivity improvement for optimization of derivatization reaction | | | | | | |
|---|---|---|---|---|---|---|
| | Ad | | NA | | DP | |
| m/z | 184.2> 166.1 | 282.3> 264.2 | 184.2> 166.1 | 282.3> 264.2 | 154> 137.0 | 350.3> 137.1 |
| *t*_{R}* | 0.635 | 2.894 | 0.54 | 3.183 | 0.666 | 3.179 |
| Not derivatized | 8119857 | | 498011 | | 305662 | |
| Heptylated | | 67421178 | | 49209671 | | 59590912 |
| Sensitivity improvement* * | 1 | 8.3 | 1 | 99 | 1 | 195 |

| | | | | | | |
|---|---|---|---|---|---|---|
| **t*_{R}: retention time (min); ** A sample not derivatized is 1. | | | | | | |

The numeric characters in Table are peak areas on SRM chromatograms.

### [Test Example 4: Detection of oxytocin in plasma]

### 1. Reagents

· An aqueous 20% formaldehyde solution
· A 3 mg/mL borane-2-picoline complex solution in isopropanol (preparation as needed)
· Acetic acid

### 2. Extraction of oxytocin from plasma

A strong cation-exchange column (Agilent, Bond Elut Plexa PCX 1 cc Cartridge 30 mg) was washed with 1% formic acid in methanol (1 mL) and was equilibrated with an aqueous 1% formic acid solution (1 mL). To an oxytocin standard (0, 20, 200 ng), pooled plasma (300 µL) was added, and the mixture was diluted with water (700 µL). The plasma dilution solution was loaded onto the column. The column was successively washed with an aqueous 1% formic acid solution (1 mL) and 1% formic acid in methanol (1 mL) and was eluted with 5% aqueous ammonia (1 mL). The eluate was concentrated with a centrifugal evaporator to give an oxytocin extract.

### 3. Derivatization

To the oxytocin extract, 50 µ of an aqueous formaldehyde solution, 50 µL of a picoline borane solution, and 10 µL of acetic acid were added, and the mixture was allowed to stand at room temperature for 30 minutes. After the derivatization, the aldehyde, the acetic acid, the boron, and the like were removed by concentration with an evaporator. After the concentration with the evaporator, the residue was dissolved in a redissolving solvent (a 30% acetonitrile solution), and the solution was subjected to LC-MS/MS analysis.

### 4. LC analysis conditions

Apparatus: Shimadzu LC-30A
Analytical column: YMC Triart C18 2.1 × 50 mm
Elution conditions: flow rate, 0.4 mL/min; solvent A, 0.1% formic acid-water; solvent B, methanol
Time (min) [solvent B%]: 0 min [0%], 3 min [0%], 3.5 min [100%], 4 min [0%], 5 min [0%]

### 5. MS/MS analysis conditions

Apparatus: Shimadzu LCMS8060 triple-quadrupole mass spectrometer
Ionization conditions: ESI positive ion mode
Collision energy (CE) and selected reaction monitoring (SRM) chromatograms are shown in Table 21.

### 6. Measurement results

The sensitivity improvement effects by N-alkylation (dimethylation) are shown in Table 21 and Table 22. The sensitivity improvement of the dimethylated product (OXT-Me₂) was 2.8 times (addition of 1 ng of OXT improved the sensitivity 2.8 times). Hence, N-alkylation improved the detection sensitivity of the peptide in plasma.

**[Table 21]**

| Table 21: SRM parameters for oxytocin (OXT) | | |
|---|---|---|
| Parent ion (M+H⁺) | m/z | CE(V) |
| OXT (not derivatized) | 1007.2>723.2 | 31 |
| OXT-Me₂ | 1035.2>751.2 | 36 |

**[Table 22]**

| Table 22: Relative sensitivity improvement of oxytocin in blood plasma | | |
|---|---|---|
| | OXT | OXT-Me₂ |
| m/z | 1007.2>723.2 | 1035.6>751.3 |
| *t*_{R}* | 3.599 | 3.622 |
| OXT 1 ng | 339 | 939 (2.8 times**) |
| OXT 10 nq | 7980.5 | 19533 (2.4 times**) |

| | | |
|---|---|---|
| **t*_{R}: retention time (min) ** Relative sensitivity improvement ratios where the sensitivity of oxytocin not derivatized is 1. | | |

The numeric characters in Table are peak areas on SRM chromatograms.

### [Test Example 5: Detection of catecholamine in urine or serum]

### 1. Reagents

· A 10% valeraldehyde50% ethanol solution in water
· A 3 mg/mL borane-2-picoline complex solution in isopropanol (preparation as needed)
· Acetic acid

### 2. Extraction of catecholamine from urine or serum

A strong cation-exchange column (Agilent, Bond Elut Plexa PCX 1 cc Cartridge 30 mg) was washed with 1% formic acid in methanol (1 mL) and was equilibrated with an aqueous 1% formic acid solution (1 mL). To a sample (1 mL of water, 1 mL of urine, or a solution prepared by diluting 300 µL of pooled serum with 700 µL of water), a catecholamine (adrenaline, noradrenaline, or dopamine) standard (0 or 200 ng) was added, and the mixture was loaded onto the column. The column was successively washed with an aqueous 1% formic acid solution (1 mL) and 1% formic acid in methanol (1 mL) and was eluted with 5% aqueous ammonia (1 mL). The eluate was concentrated with a centrifugal evaporator to give a catecholamine extract.

### 3. Derivatization

To the catecholamine extract, 50 µL of an aqueous valeraldehyde solution, 50 µL of a picoline borane solution, and 10 µL of acetic acid were added, and the mixture was allowed to stand at room temperature for 30 minutes. After the derivatization, the aldehyde, the acetic acid, the boron, and the like were removed by concentration with an evaporator. After the concentration, the residue was dissolved in a redissolving solvent (a 30% acetonitrile solution), and the solution was subjected to LC-MS/MS analysis.

### 4. LC analysis conditions

Apparatus: Shimadzu LC-20A
Analytical column: OSAKA SODA CAPCELL CORE C18 2.1 × 75 mm
Elution conditions: flow rate, 0.4 mL/min; solvent A, 0.1% formic acid-water; solvent B, methanol
Time (min) [solvent B%]: 0 min [0%], 0.5 min [0%], 2 min [100%], 3 min [100%], 3.5 min [0%], 5 min [0%]

### 5. MS/MS analysis conditions

Apparatus: Shimadzu LCMS8040 triple-quadrupole mass spectrometer
Ionization conditions: ESI positive ion mode
Collision energy (CE) and selected reaction monitoring (SRM) chromatograms are shown in Table 23.

### 6. Measurement results

The sensitivity improvement effects by N-alkylation (valeration) are shown in Table 23 to Table 26. Even in urine or serum, the derivatization improved the detection sensitivity.

**[Table 23]**

| Table 23: SRM parameters for catecholamines | | | |
|---|---|---|---|
| 1. SRM parameters for adrenaline (Ad) | | | |
| Parent ion (M+H⁺) | m/z | | CE(V) |
| Ad (not derivatized) | 184.2>107.0 | | 22 |
| Ad-V | 254.3>236.2 | | 14 |
| Ad-V | 254.3>166.1 | | 23 |
| Ad-V | 254.3>137.0 | | 23 |

| 2. SRM parameters for noradrenaline (NA) | | | |
|---|---|---|---|
| Parent ion (M+H⁺) | m/z | CE(V) | |
| NA (not derivatized) | 170.0>107.1 | 20 | |
| NA-V₂ | 310.3>292.2 | 18 | |
| NA-V₂ | 310.3>222.1 | 25 | |
| NA-V₂ | 310.3>137.1 | 28 | |

| 3. SRM parameters for dopamine (DP) | | | |
|---|---|---|---|
| Parent ion (M+H⁺) | m/z | | CE(V) |
| DP (not derivatized) | 154.0>91.1 | | 25 |
| DP-V₂ | 294.2>137.0 | | 23 |
| DP-V₂ | 294.2>119.1 | | 35 |
| DP-V₂ | 294.2>91.0 | | 48 |

**[Table 24]**

| Table 24: Relative sensitivity improvement of adrenaline from urine or serum | | | | |
|---|---|---|---|---|
| | Not derivatized | | Derivatized | |
| m/z | 184.2>107.0 | 254.3>236.2 | 254.3>166.1 | 254.3>236.2 |
| *t*_{R}* | 0.614 | 2.670 | 2.670 | 2.669 |
| Ad (urine) | 7761 | 7325718 | 1170643 | 1145893 |
| Ad-V (serum) | N D** | 68294 | 11262 | 15683 |
| Sensitivity improvement (urine)*** | 1 | 944 | 151 | 148 |

| | | | | |
|---|---|---|---|---|
| **t*_{R}: retention time (min); **ND: not determined *** Relative sensitivity improvement ratios where the sensitivity of adrenaline not derivatized is 1. | | | | |

The numeric characters in Table are peak areas on SRM chromatograms.

**[Table 25]**

| Table 25: Relative sensitivity improvement of noradrenaline from urine or serum | | | | |
|---|---|---|---|---|
| | Not derivatized | | Derivatized | |
| m/z | 170.0>107.1 | 310.3>292.2 | 310.3>222.2 | 310.3>137.1 |
| *t*_{R}* | 0.583 | 3.020 | 3.013 | 3.011 |
| NA (urine) | 259174 | 7946132 | 1045341 | 1948453 |
| NA-V₂ (serum) | N D** | 86057 | 11547 | 21750 |
| Sensitivity improvement (urine)*** | 1 | 31 | 4 | 8 |

| | | | | |
|---|---|---|---|---|
| **t*_{R}: retention time (min); **ND: not determined *** Relative sensitivity improvement ratios where the sensitivity of dopamine not derivatized is 1. | | | | |

The numeric characters in Table are peak areas on SRM chromatograms.

**[Table 26]**

| Table 26: Relative sensitivity improvement of dopamine from urine or serum | | | | |
|---|---|---|---|---|
| | Not derivatized | | Derivatized | |
| m/z | 154.0>91.1 | 294.2>137.0 | 294.2>119.1 | 294.2>91.0 |
| *t*_{R}* | 0.670 | 3.034 | 3.027 | 3.031 |
| DP (urine) | 57653 | 1924018 | 656209 | 1645208 |
| DP-V₂ (serum) | ND** | 47772 | 638 | 17641 |
| Sensitivity improvement (urine) *** | 1 | 33 | 11 | 29 |

| | | | | |
|---|---|---|---|---|
| **t*_{R}: retention time (min); **ND: not determined *** Relative sensitivity improvement ratios where the sensitivity of dopamine not derivatized is 1. The numeric characters in Table are peak areas on SRM chromatograms. | | | | |

In the present description, numbers or letters such as "first, second, third, ..." "A, B, C, ...", and "first-stage, second-stage, third-stage, ..." may be used for simple explanations, but this does not limit the present invention to a narrow interpretation of the order or the like, and the order may be changed appropriately. In the present description, for example, "doing (of doing)" in "derivatizing (of derivatizing)" or the like may mean a method, process, means, step, or the like, and these terms may be used interchangeably as appropriate. For example, "step" may also be used as "doing (of doing)", a method, process, means, or the like; "process" may also be used as "doing (of doing)", a method, step, means, or the like; "means" may also be used as "doing (of doing)", a method, process, step, mechanism, system, apparatus, or part; and "part" may also be used as a mechanism, means, apparatus, or system, or as a mechanism, means, or apparatus, or the like to be included therein. A combination product may be a combination article or a combination.

## Claims

1. A derivatization reagent comprising a compound represented by Formula (1):
RR'CO (1)
[in Formula (1), R and R' are each independently a hydrogen atom, a substituted or
unsubstituted alkyl group having 1 to 11 carbon atoms, or a substituted or unsubstituted aryl group having 1 to 11 carbon atoms].

2. The derivatization reagent according to claim 1, used to N-alkylate or N-arylate an amino group of a nitrogen-containing compound.

3. The derivatization reagent according to claim 2, wherein the nitrogen-containing compound is at least one compound selected from an amino acid, a peptide, and a catecholamine contained in a biological substance.

4. The derivatization reagent according to claim 1, wherein the derivatization reagent is used to N-alkylate or N-arylate an amino group of a nitrogen-containing compound, and
(a) when the nitrogen-containing compound is an amino acid or a peptide in a biological substance, R and R' in Formula (1) are each independently a hydrogen atom or a substituted or unsubstituted alkyl group having 1 to 8 carbon atoms, or
(b) when the nitrogen-containing compound is a catecholamine in a biological substance, R and R' in Formula (1) are each independently a substituted or unsubstituted alkyl group having 2 to 9 carbon atoms.

5. The derivatization reagent according to claim 1 or 2, used in mass spectrometry.

6. A derivatization reagent kit comprising:
the derivatization reagent according to any one of claims 1 to 4; and
a reducing agent.

7. The derivatization reagent kit according to claim 6, wherein the reducing agent comprises at least one selected from a borane, a complex containing a borane, and a salt of a borohydride compound.

8. A method for improving sensitivity of a nitrogen-containing compound in mass spectrometry, the method comprising derivatization of N-alkylating or N-arylating an amino group of a nitrogen-containing compound with the derivatization reagent kit according to claim 6 into a secondary amine or a tertiary amine.
